# EUROPEAN PATENT APPLICATION

(11) **EP 3 255 137 A1**
(43) Date of publication of application: **13.12.2017**
(21) Application number: 16746694.5
(22) Date of filing: 04.02.2016
(51) Int. Cl.: C12M 1/00

(54) **CELL CULTURE SYSTEM**

(30) Priority: 05.02.2015 JP 2015020798; 23.07.2015 JP 2015145829
(71) Applicant: OLYMPUS CORPORATION, Hachioji-shi Tokyo 192-8507 (JP)
(72) Inventor: KIMURA, Hiroyuki, Tokyo 192-8507 (JP); MINAMI, Tatsuya, Tokyo 192-8507 (JP); MAKARA, Yasunori, Tokyo 192-8507 (JP)
(74) Representative: Schicker, Silvia
(86) International application number: PCT/JP2016/053381
(87) International publication number: WO 2016/125863

(57) **Abstract**

Provided is a cell-culturing apparatus (100) including a culturing unit (1) in which a culturing vessel (3) is placed and an incubator (2) in which a plurality of culturing units (1) are placed in an interior thereof, and that can maintain the interior thereof at an environment that is appropriate for cell culturing, wherein the incubator (2) includes openings (5) that are provided for each of the plurality of culturing units (1) and through which the culturing units (1) can separately be moved into and out of the incubator (2), and closing means that are provided at the openings (5) and that seals off the internal environment of the incubator (2) from outside when the culturing unit (1) is taken out of the incubator (2).

## Description

### {Technical Field}

The present invention relates to a cell-culturing apparatus for culturing cells in a cell-culturing vessel.

### {Background Art}

In recent years, in association with the development in research on regenerative medicine, bio-pharmaceuticals, and the like, there is a demand for preparing a large quantity of cells. Cells are normally cultured in an incubator (warm bath) in which it is possible to maintain an environment that is appropriate for growing them. In order to regularly observe the state of the cells and to allow the medium to be exchanged, it is necessary to perform such procedures by taking samples such as cells or the like out of the incubator (for example, see Patent Literature 1). When moving the samples such as cells or the like into and out of the incubator, the environment (temperature, humidity, CO₂ concentration, and so forth) in the incubator is exposed to the external environment, which temporarily disturbs the steady state, and thus, the samples such as cells or the like in the incubator may experience stress.

On the other hand, the samples taken out of the incubator are also exposed to the external environment, which disturbs the steady state while performing procedures, and thus, the samples experience stress.

### {Citation List}

### {Patent Literature}

{PTL 1} Japanese Unexamined Patent Application, Publication No. 2010-273603

### {Summary of Invention}

### {Technical Problem}

Therefore, the procedures involved in moving the samples such as cells or the like into and out of the incubator need to be performed by taking as little time as possible. However, for a worker who handles a large quantity of samples, it is extremely difficult to move the samples into and out of the incubator without disturbing the environment in the incubator. Furthermore, for example, because preparing cells for clinical usage requires procedures to be carried out in a closed system by using an isolator or the like, the movable space for the worker is limited, and thus, the burden on the worker is further increased.

In addition, although it is preferable that the samples taken out of the incubator be retuned to the incubator as soon as possible, doing so is difficult for a worker who handles a large quantity of samples.

The present invention has been conceived in light of the above-described circumstances, and an object thereof is to provide a cell-culturing apparatus with which it is possible to decrease the changes in the internal environment in an incubator by simplifying the procedures involved when moving samples such as cells or the like into and out of the incubator.

An additional object is to provide a cell-culturing apparatus with which it is possible to maintain the samples taken out of the incubator in an environment that is similar to the environment in the incubator.

### {Solution to Problem}

In order to achieve the above-described objects, the present invention provides the following solutions.

An aspect of the present invention is a cell-culturing apparatus including: a culturing unit in which a culturing vessel is placed; and an incubator in which a plurality of culturing units are placed in an interior thereof, and that can maintain the interior thereof at an environment that is appropriate for cell culturing, wherein the incubator includes openings that are provided for each of the plurality of culturing units and through which the culturing units can separately be moved into and out of the incubator, and closing means that are provided at the openings and that seal off the internal environment of the incubator from outside when the culturing units are taken out of the incubator.

With this aspect, the worker can take only a desired sample out of the incubator and accommodate it in the incubator, and it is possible to reduce changes in the environment in the incubator when moving the sample into and out of the incubator.

The above-described aspect may be further provided with a driving means that moves the incubator.

In addition, the driving means may be a means for moving the incubator in the direction in which the culturing units are arranged in the interior of the incubator.

By doing so, the worker can move a desired sample into and out of the incubator at a position at which handling thereof is easier.

1 In the above-described aspect, the culturing unit may be provided with a caster.

By doing so, the worker can more easily move the culturing unit into and out of the incubator, and the culturing unit can easily be moved outside the incubator.

In the above-described aspect, the culturing unit may be provided with a solution supplying means that supplies a solution such as a medium or the like to the culturing vessel and a solution discharging means that discharges a waste liquid from the culturing vessel. By doing so, it is possible to replenish the solution such as a medium or the like and to discard the waste liquid in a simple manner.

The plurality of culturing units may be arrayed in the interior of the incubator in a horizontal direction; the plurality of culturing units may be arrayed in the interior of the incubator in a vertical direction; and the plurality of culturing units may be arrayed in the interior of the incubator in the horizontal direction and the vertical direction.

Another aspect of the present invention is a cell-culturing apparatus including: a culturing unit in which a culturing vessel is placed; an incubator in which the culturing unit is placed in an interior thereof, and that can maintain the interior thereof at an environment that is appropriate for cell culturing; a work space in which the culturing unit taken out of the incubator is placed; a gas-adjusting means that adjusts at least one of gas composition, gas temperature, and gas humidity; and a gas-flowing means that makes a gas flow between the culturing unit placed in the work space and the gas-adjusting means.

With this aspect, because the worker can take only a desired sample out of the incubator and accommodate it in the incubator, and because he/she can maintain the gas environment of the taken-out sample steady, it is possible to reduce the changes in the environment as compared with that in the incubator, and thus, it is possible to reduce the stress experienced by the sample.

In the above-described aspect, the incubator may serve as the gas-adjusting means. By doing so, because it is not necessary to separately install the gas-adjusting means, the apparatus becomes compact, and it is also advantageous in terms of costs.

In the above-described aspect, the culturing unit may form an open space in the culturing unit when being placed in the interior of the incubator, and may form a closed space in the culturing unit when being placed in the interior of the work space. By doing so, it is possible to maintain the internal environment of the culturing unit so as to be the same as the internal environment of the incubator both when inside and outside the incubator, and it is possible to reduce the stress experienced by the sample due to changes in the environment.

In the above-described aspect, the gas-flowing means may make gas circulate between the culturing unit placed in the work space and the gas-adjusting means. By doing so, it is possible to efficiently use the adjusted gas, and thus, it is possible to reduce the costs involved in adjusting the gas.

### {Advantageous Effects of Invention}

The present invention affords an advantage in that, it is possible to suppress changes in the environment in an incubator to minimum necessary level because it is possible for a worker to move only a desired sample into and out of the incubator in a simple manner, and thus, it is possible to reduce the influences on the sample. In addition, for example, in the case in which procedures are performed in a closed environment by using an isolator or the like, the efficiency of procedures performed by a worker is enhanced because the procedures involved in moving the sample into and out of the incubator is simplified.

### {Brief Description of Drawings}

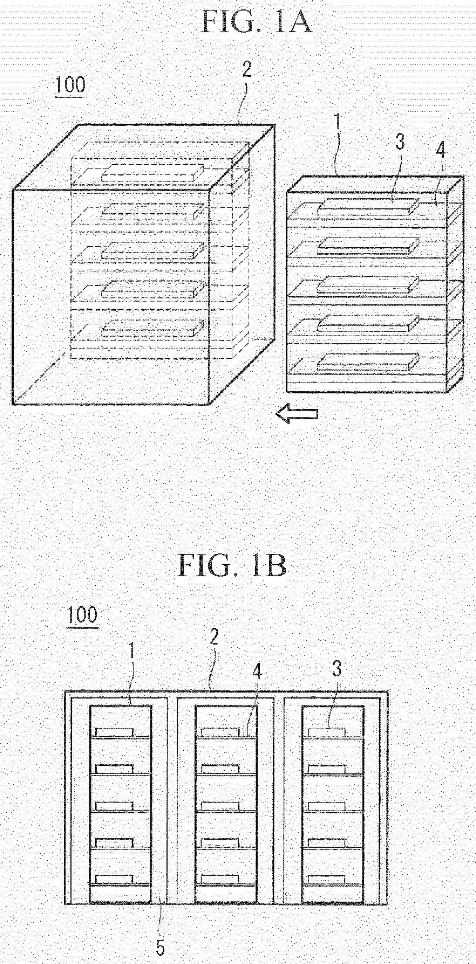

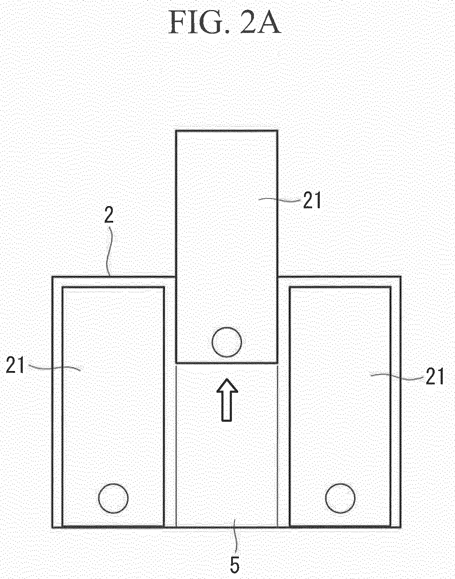

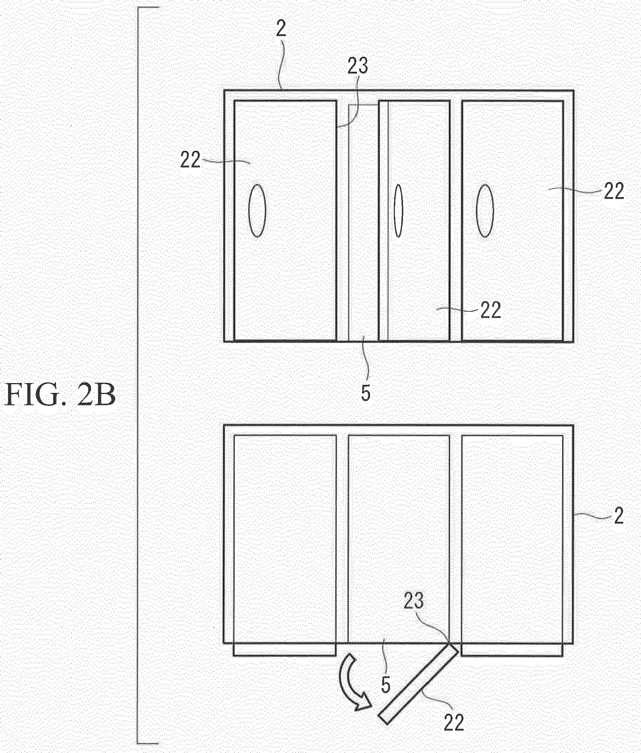

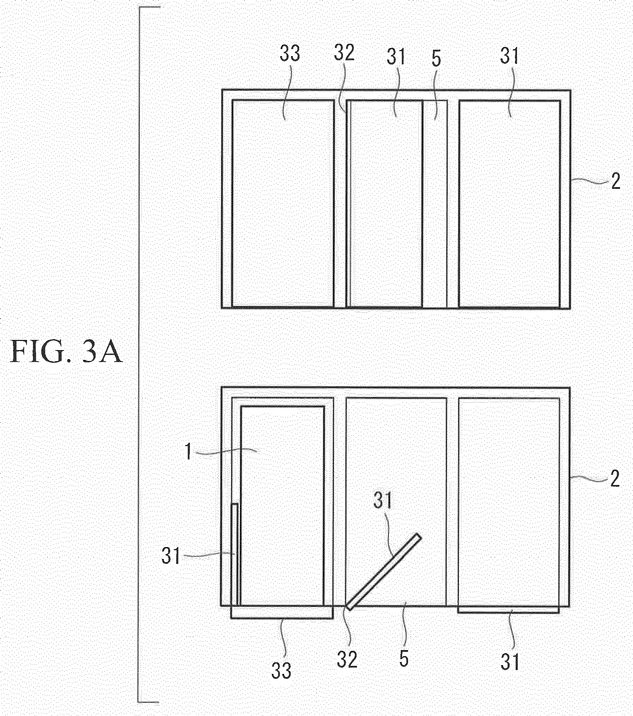

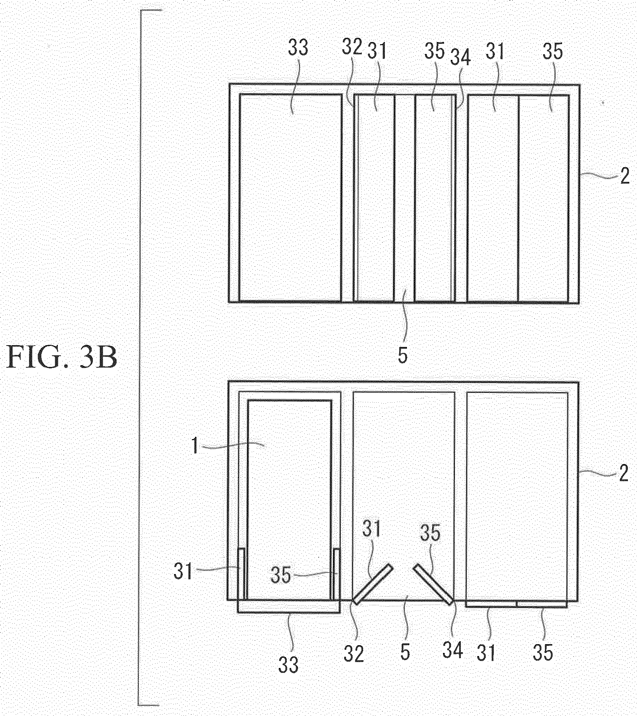

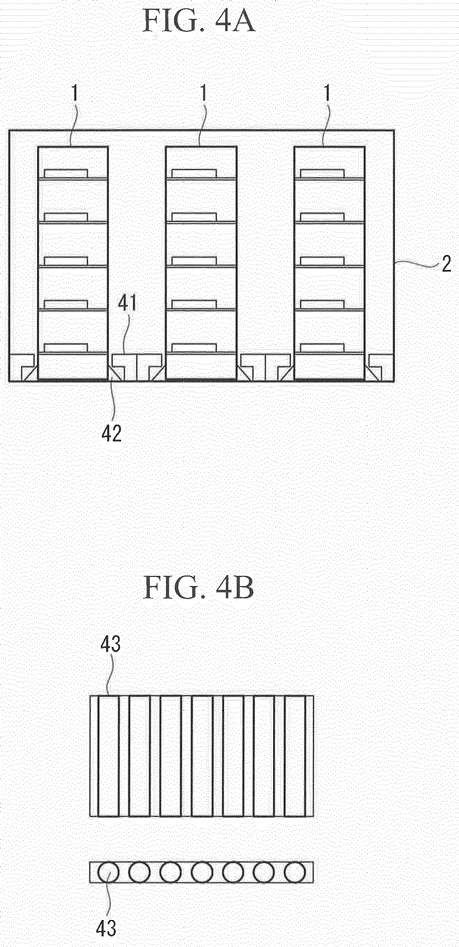

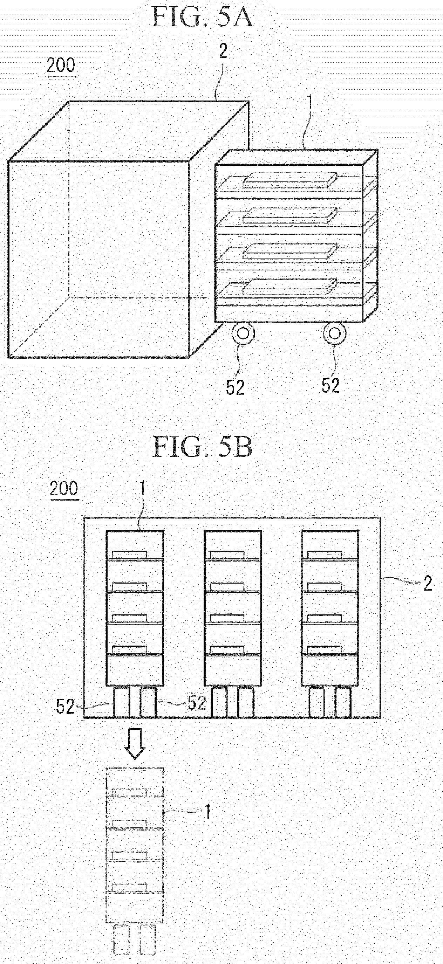

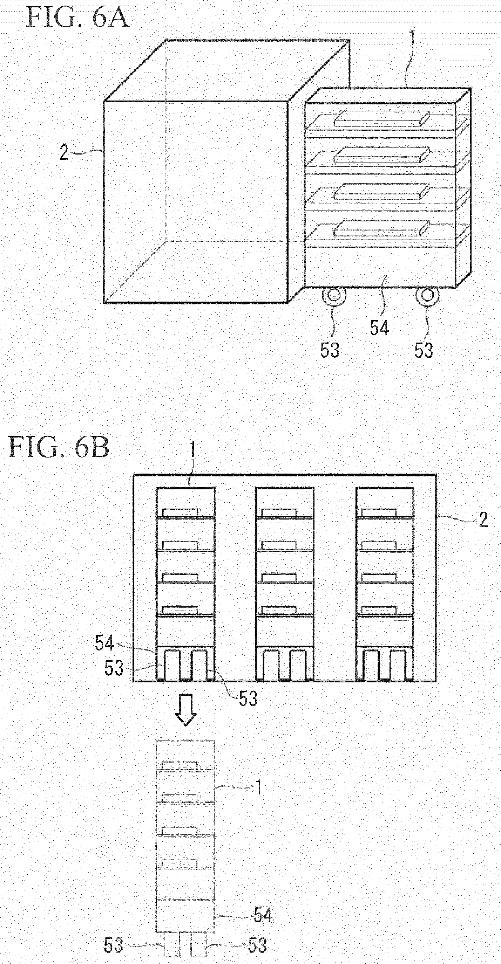

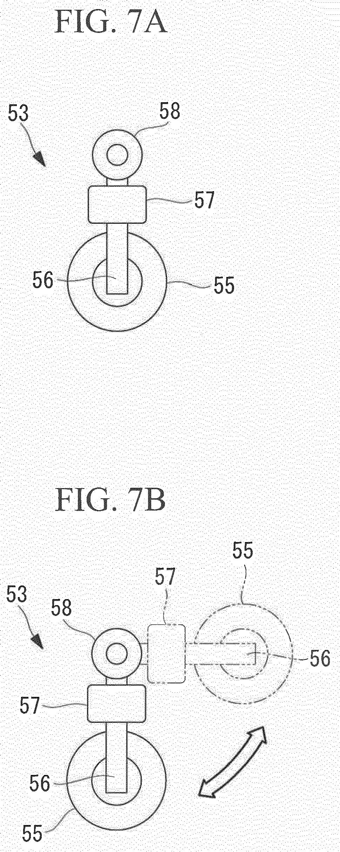

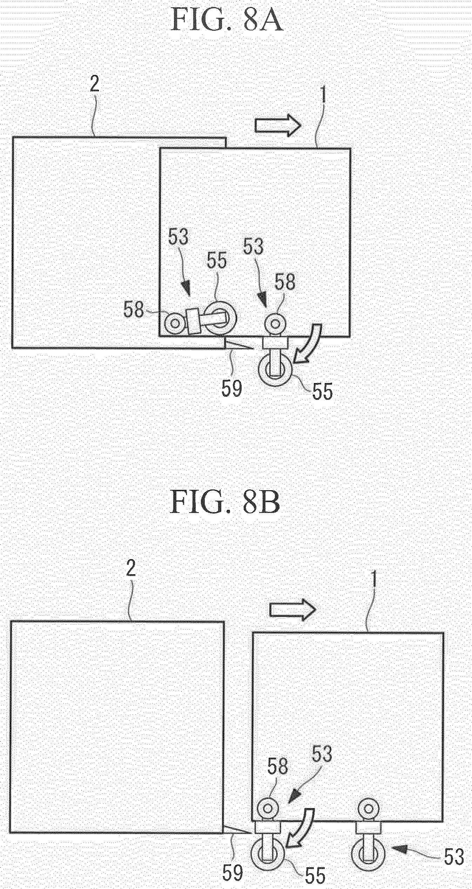

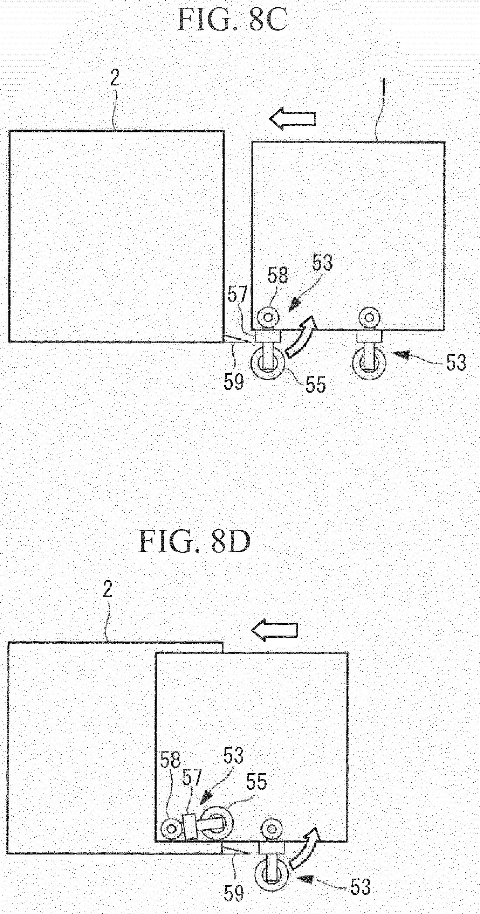

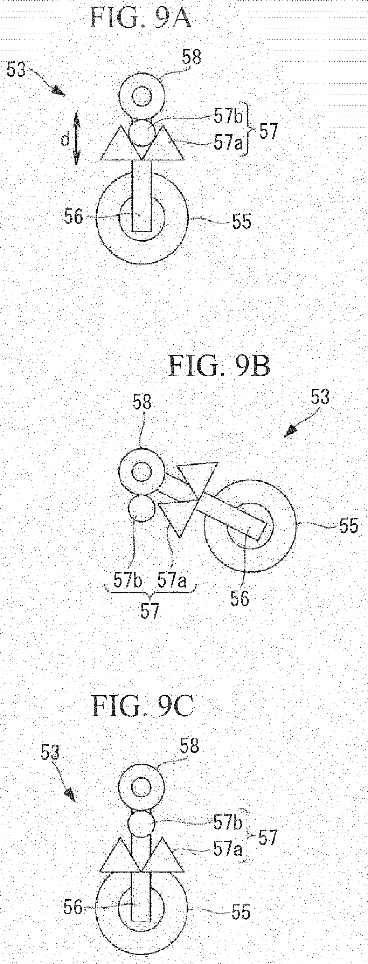

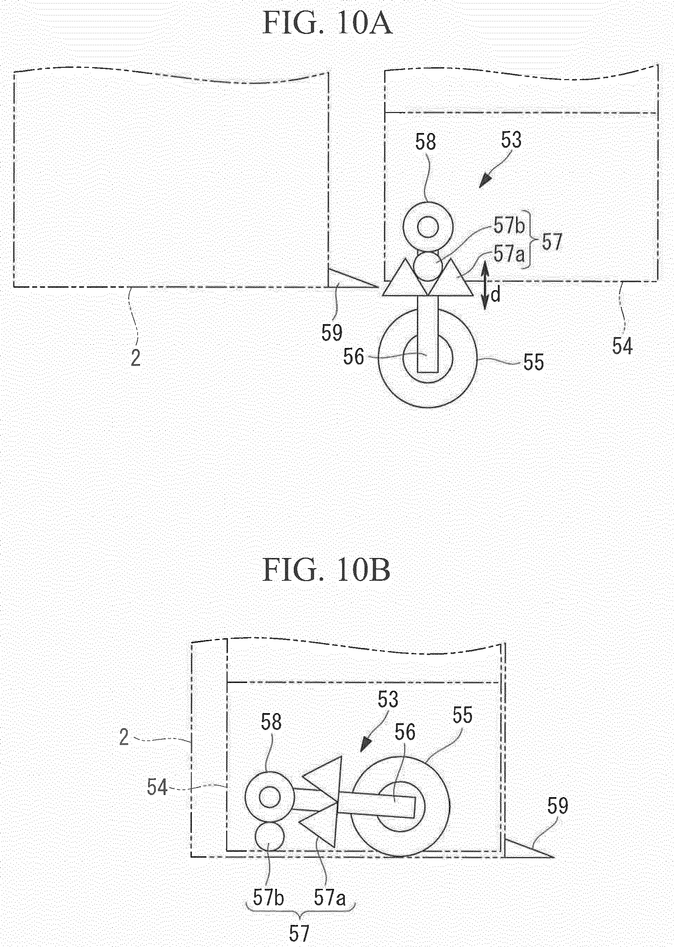

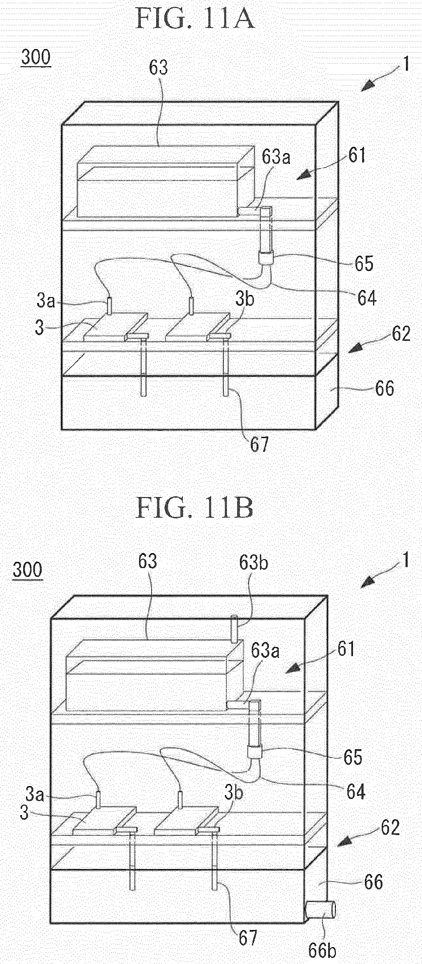

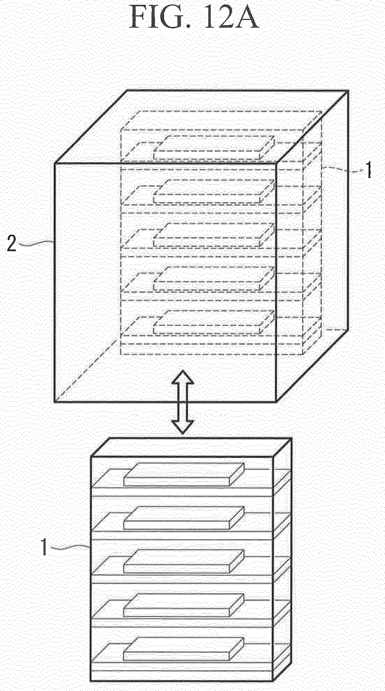

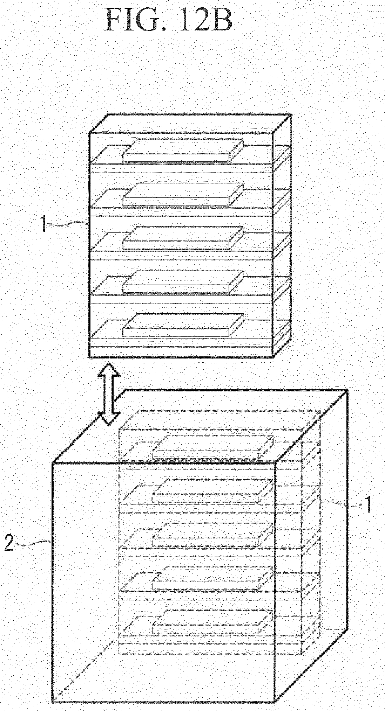

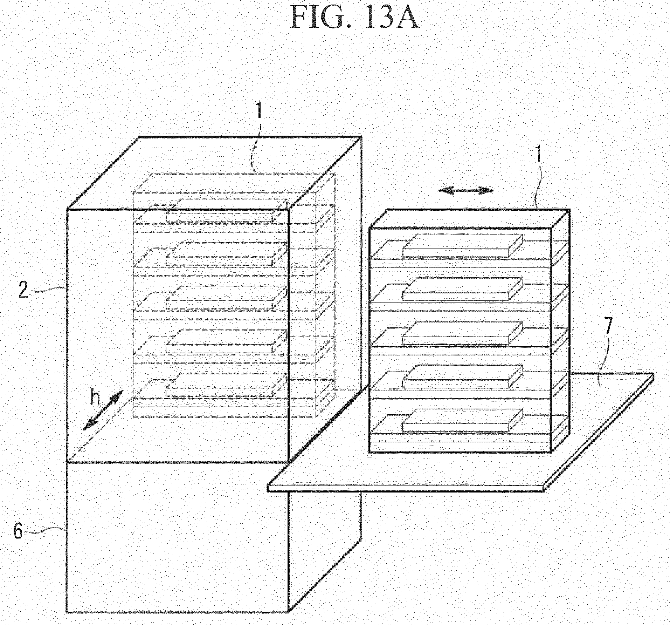

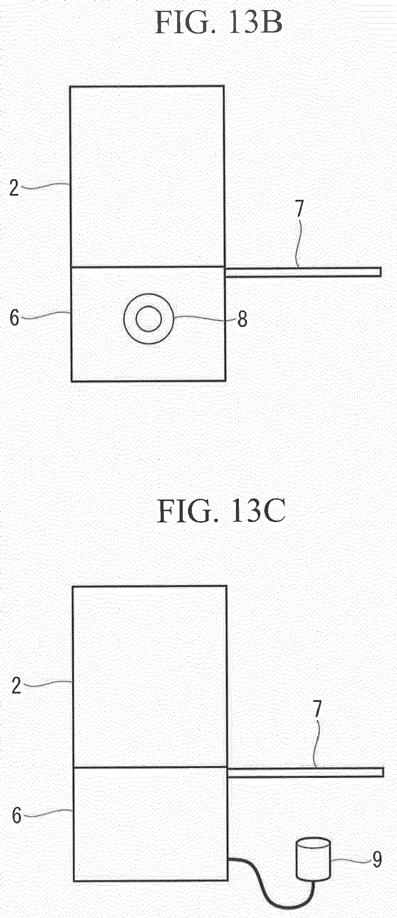

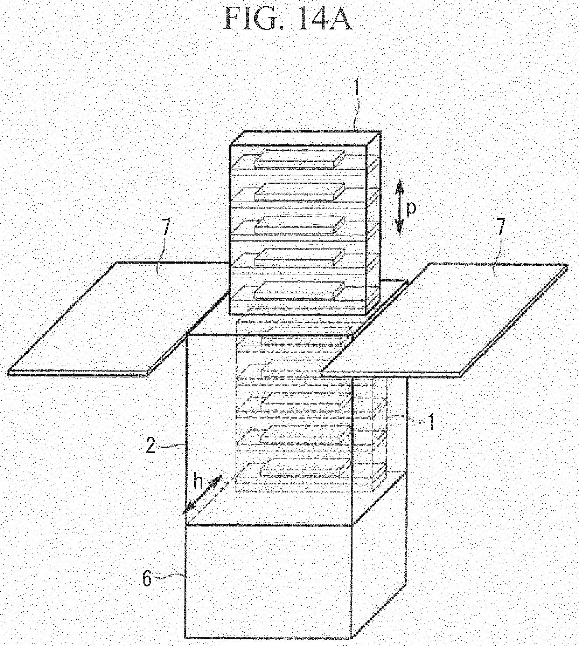

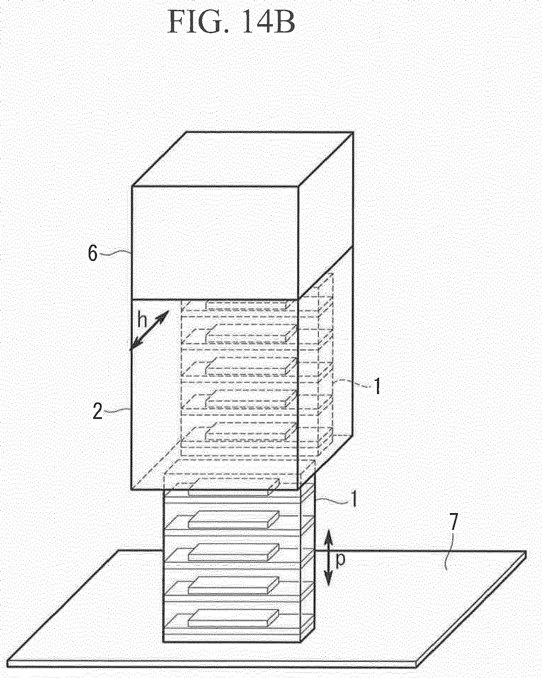

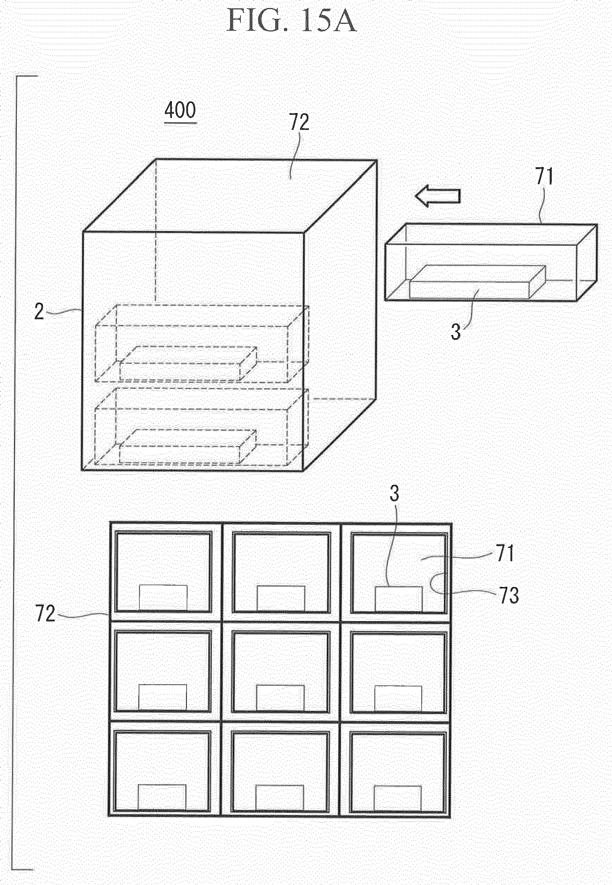

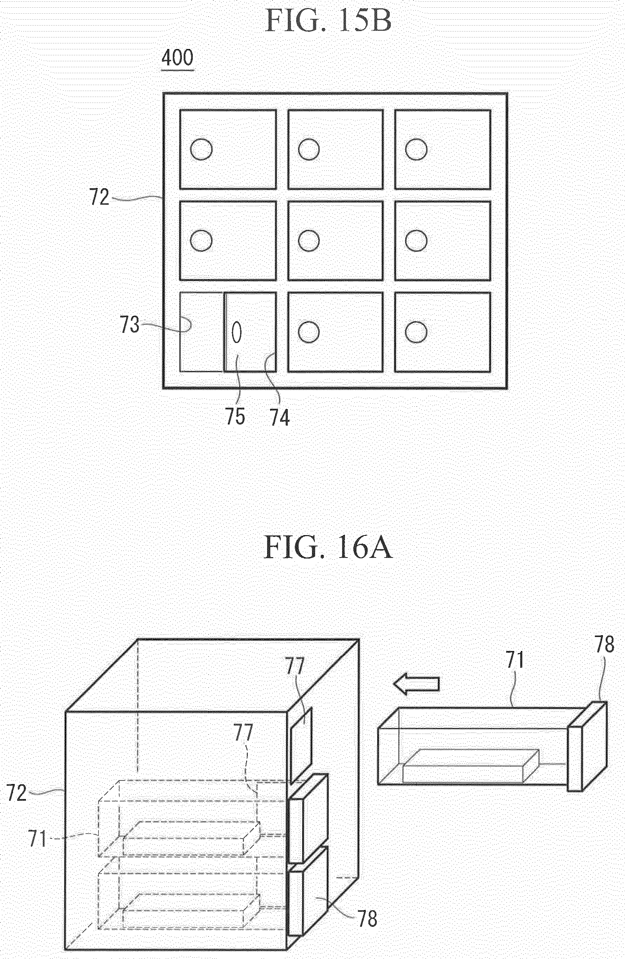

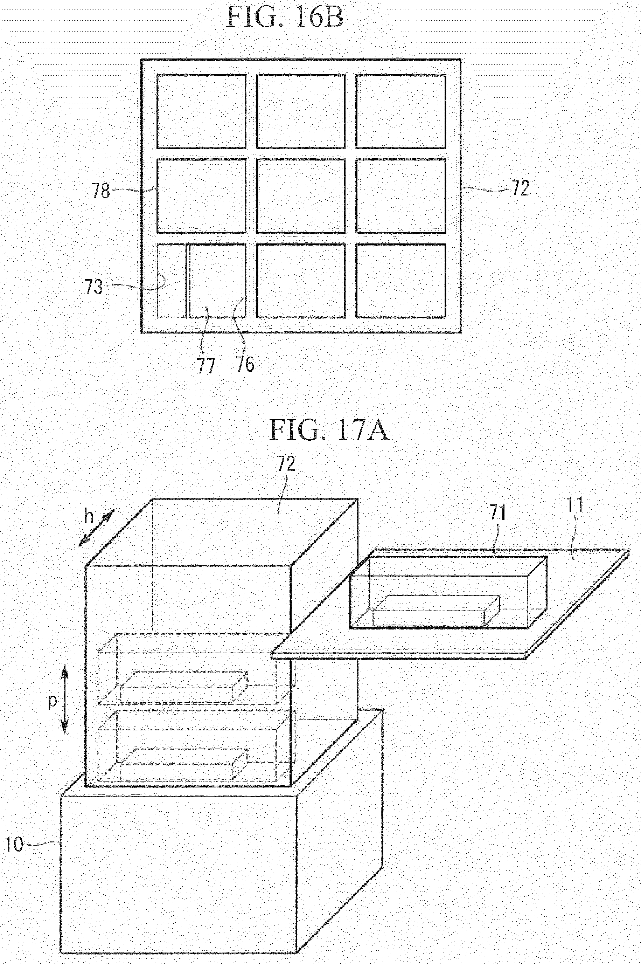

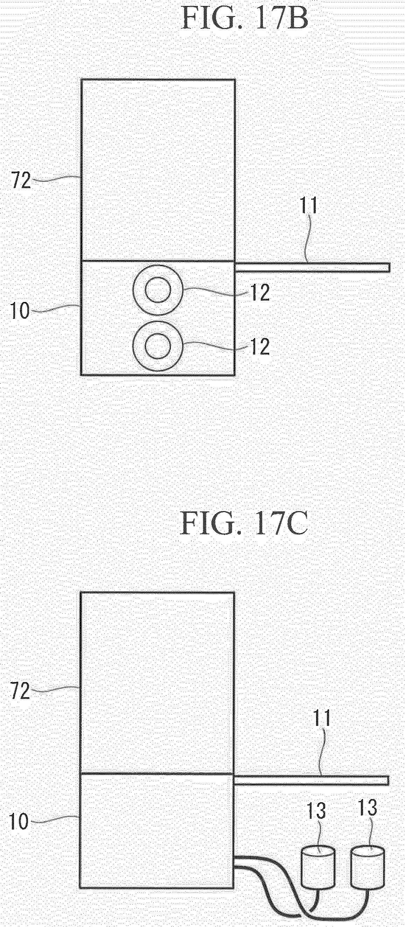

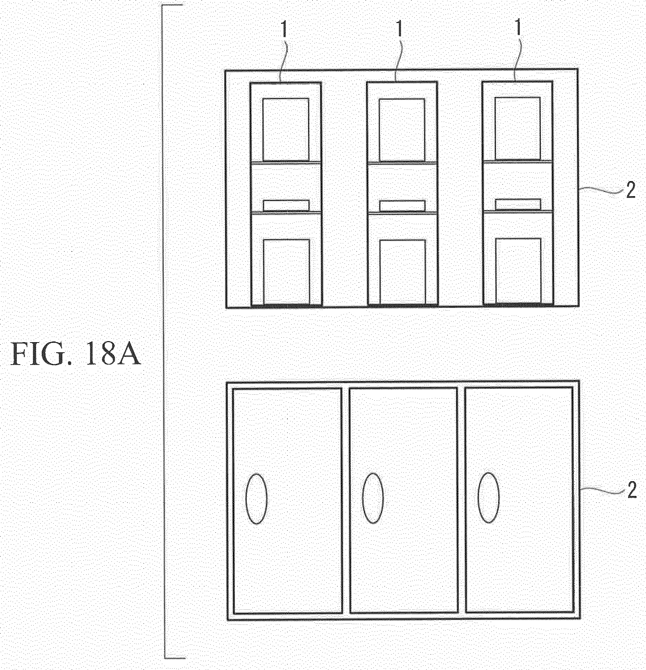

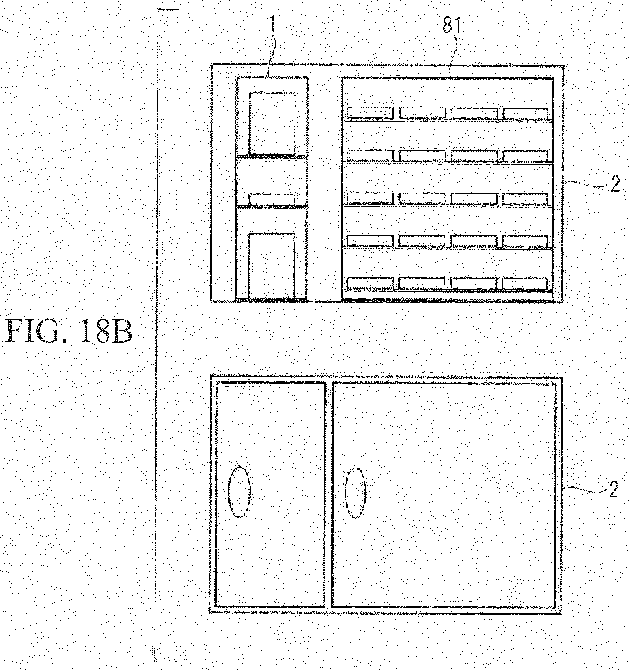

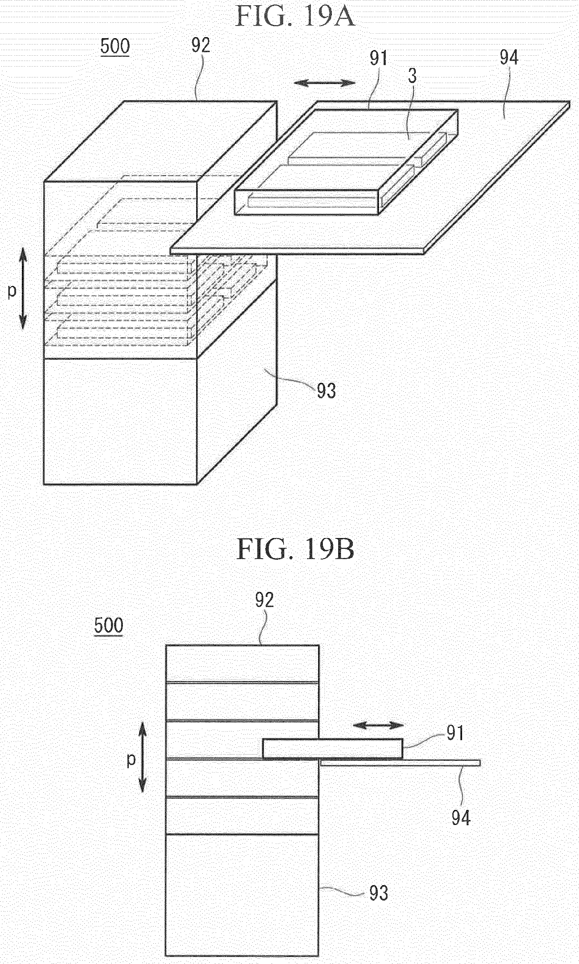

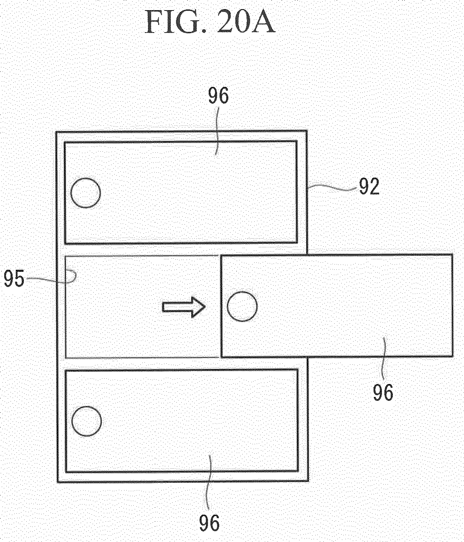

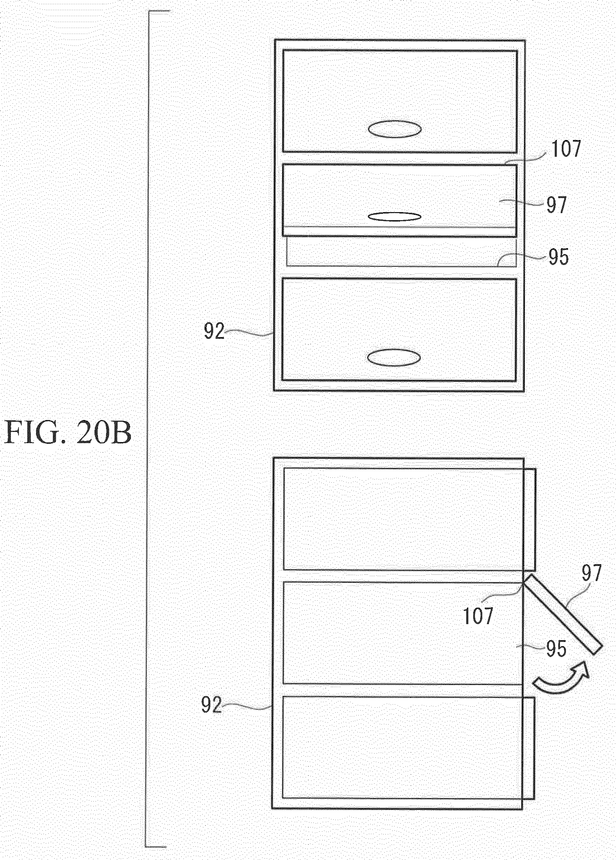

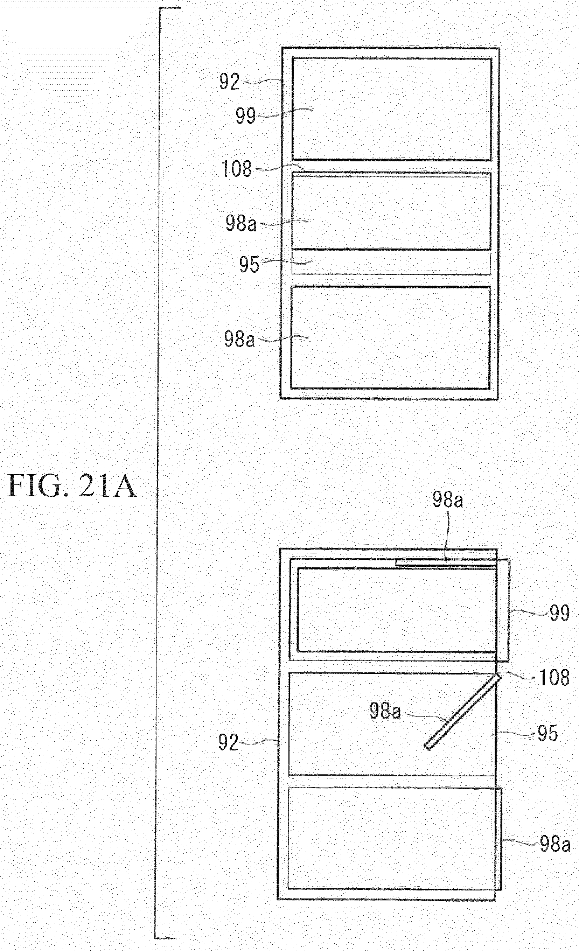

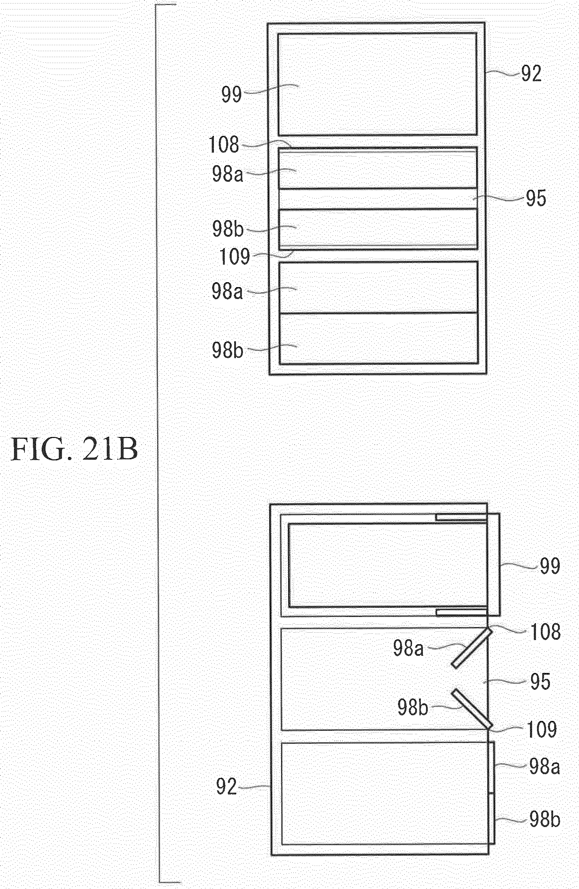

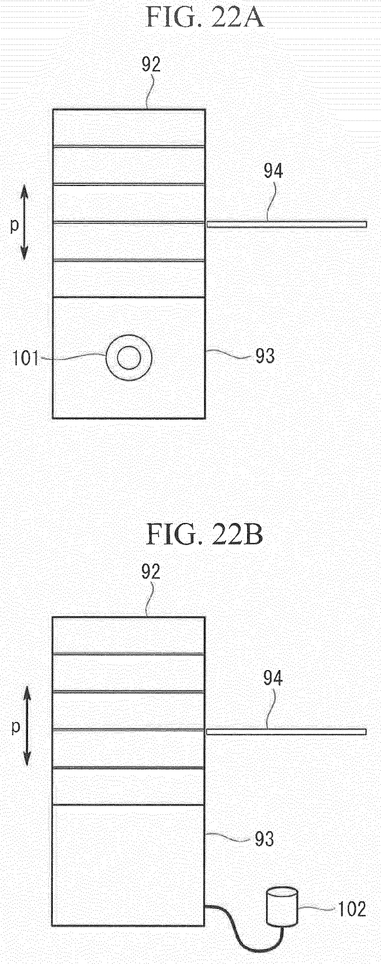

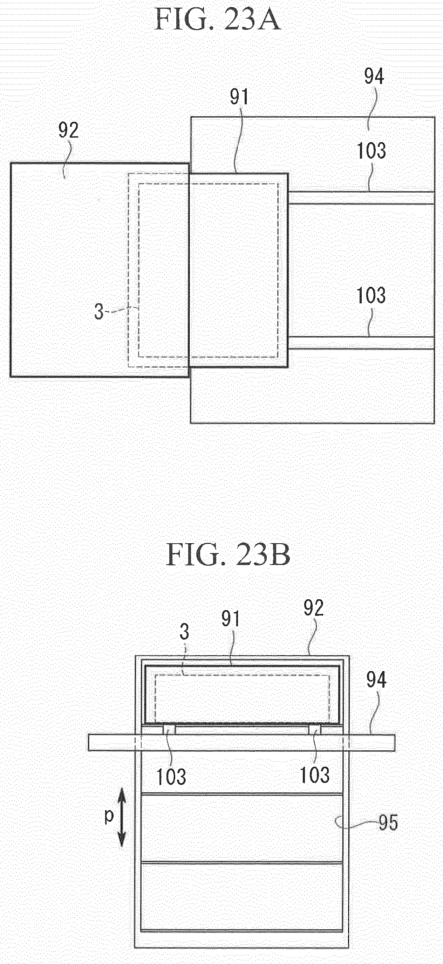

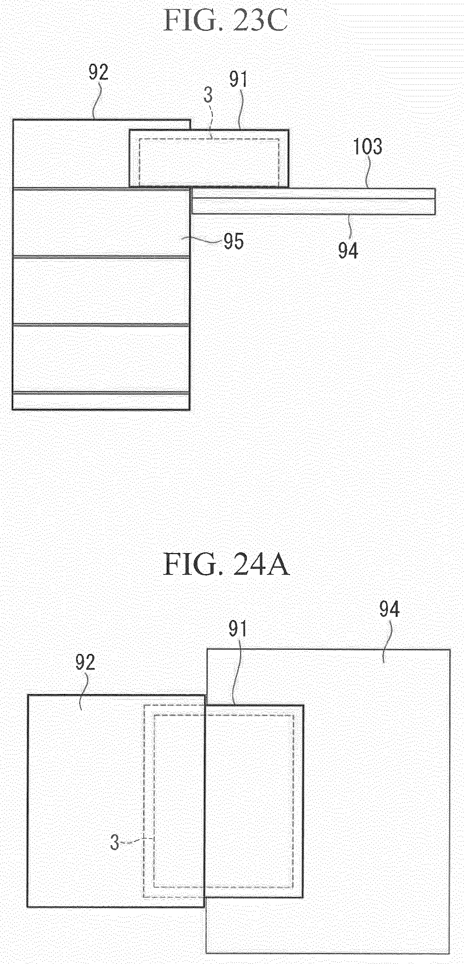

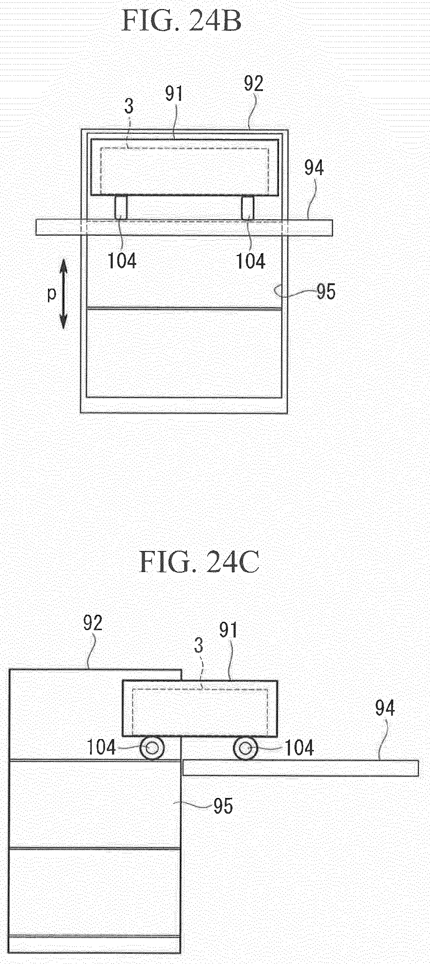

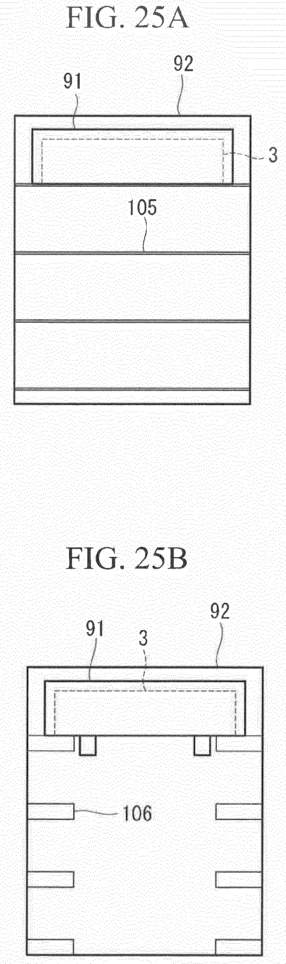

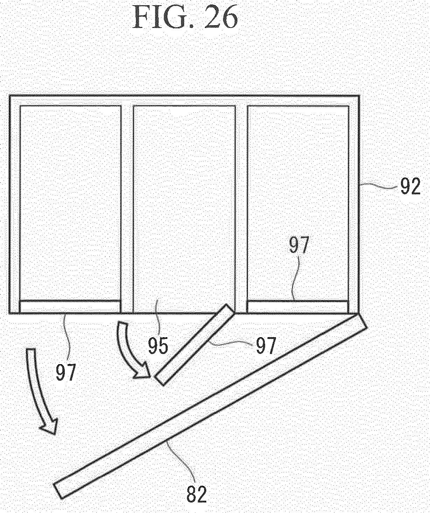

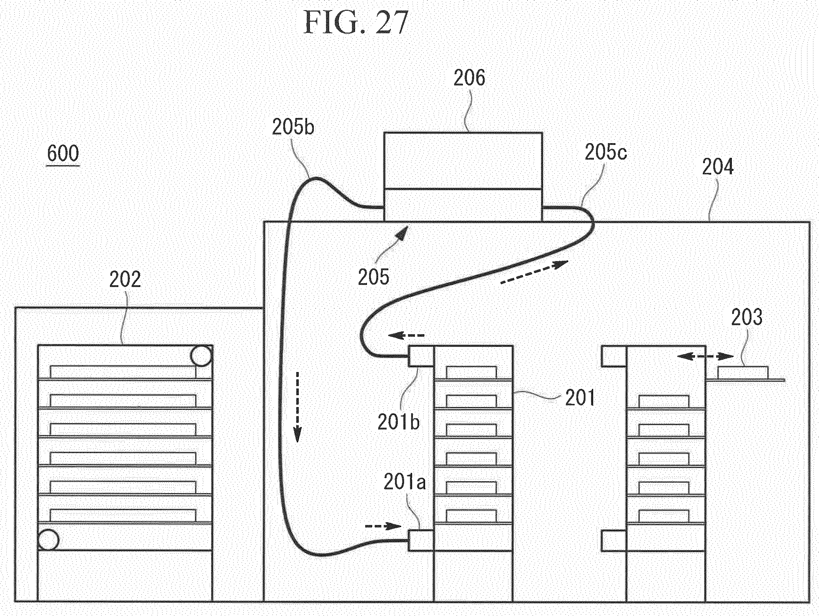

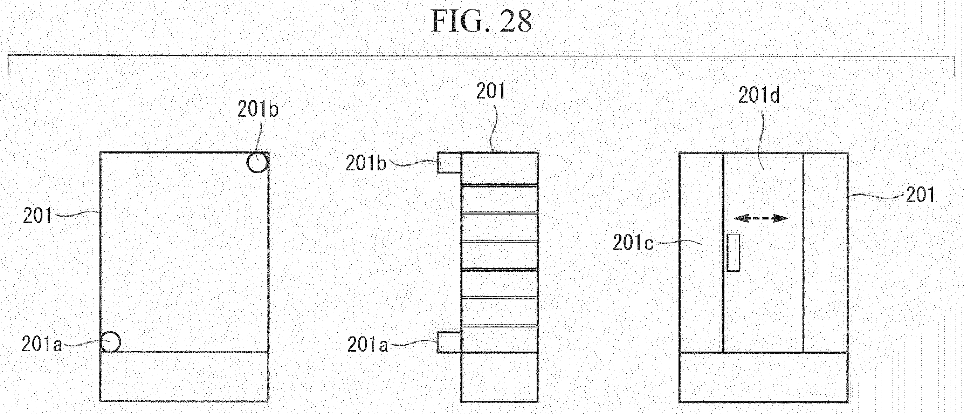

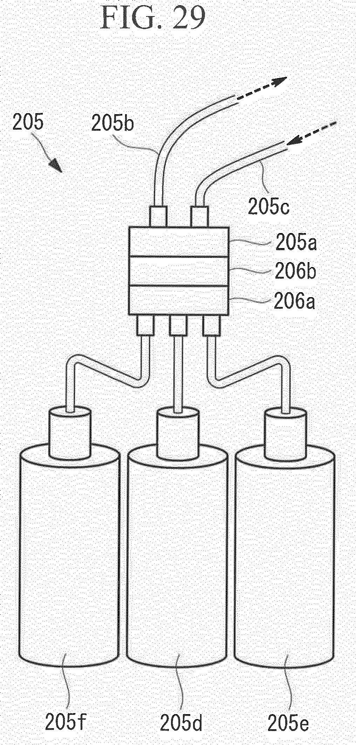

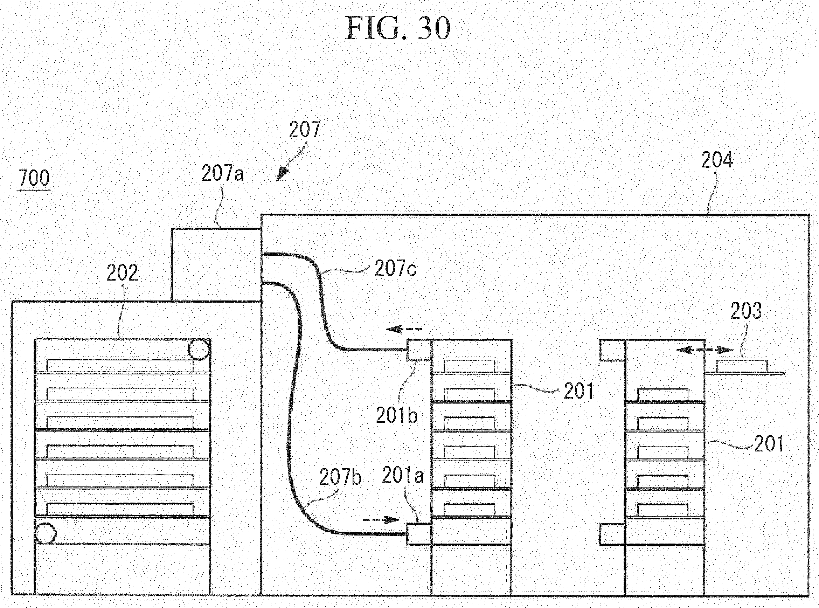

{Fig. 1A} Fig. 1A is an explanatory diagram showing, in outline, the configuration of a cell-culturing apparatus according to a first embodiment of the present invention.
{Fig. 1B} Fig. 1B is a front view of the cell-culturing apparatus in Fig. 1A.
{Fig. 2A} Fig. 2A is an explanatory diagram showing, in outline, the configuration of an example closing means of the present invention.
{Fig. 2B} Fig. 2B is an explanatory diagram showing, in outline, the configuration of another example closing means of the present invention.
{Fig. 3A} Fig. 3A is an explanatory diagram showing, in outline, the configuration of an example closing means of the present invention.
{Fig. 3B} Fig. 3B is an explanatory diagram showing, in outline, the configuration of another example closing means of the present invention.
{Fig. 4A} Fig. 4A is an explanatory diagram showing, in outline, the configuration of a rail member of the present invention.
{Fig. 4B} Fig. 4B is an explanatory diagram showing, in outline, the configuration of a movement facilitating means of the present invention.
{Fig. 5A} Fig. 5A is an explanatory diagram showing, in outline, the configuration of a cell-culturing system according to a second embodiment of the present invention.
{Fig. 5B} Fig. 5B is a front view of the cell-culturing system in Fig. 5A.
{Fig. 6A} Fig. 6A is an explanatory diagram showing, in outline, the configuration of the cell-culturing system according to the second embodiment of the present invention.
{Fig. 6B} Fig. 6B is an explanatory diagram showing, in outline, the configuration of the cell-culturing system according to the second embodiment of the present invention.
{Fig. 7A} Fig. 7A is an explanatory diagram showing, in outline, the configuration of an example rolling means of the present invention.
{Fig. 7B} Fig. 7B is a diagram for explaining the operation of the rolling means in Fig. 7A.
{Fig. 8A} Fig. 8A is an explanatory diagram showing, in outline, the configuration of an example rolling means of the present invention.
{Fig. 8B} Fig. 8B is an explanatory diagram showing, in outline, the configuration of the example rolling means of the present invention.
{Fig. 8C} Fig. 8C is an explanatory diagram showing, in outline, the configuration of the example rolling means of the present invention.
{Fig. 8D} Fig. 8D is an explanatory diagram showing, in outline, the configuration of the example rolling means of the present invention.
{Fig. 9A} Fig. 9A is an explanatory diagram showing, in outline, the configuration of an example rolling means of the present invention.
{Fig. 9B} Fig. 9B is an explanatory diagram showing, in outline, the configuration of the example rolling means of the present invention.
{Fig. 9C} Fig. 9C is an explanatory diagram showing, in outline, the configuration of the example rolling means of the present invention.
{Fig. 10A} Fig. 10A is an explanatory diagram showing, in outline, the configuration of an example rolling means of the present invention.
{Fig. 10B} Fig. 10B is a diagram for explaining the operation of the rolling means in Fig. 10A.
{Fig. 11A} Fig. 11A is an explanatory diagram showing, in outline, the configuration of a cell-culturing apparatus according to a third embodiment of the present invention.
{Fig. 11B} Fig. 11B is an explanatory diagram showing, in outline, the configuration of a modification of the cell-culturing apparatus in Fig. 11A.
{Fig. 12A} Fig. 12A is an explanatory diagram showing, in outline, the configuration of a modification of the cell-culturing apparatuses according to the first to third embodiments of the present invention.
{Fig. 12B} Fig. 12B is an explanatory diagram showing, in outline, the configuration of another modification of the cell-culturing apparatuses according to the first to third embodiments of the present invention.
{Fig. 13A} Fig. 13A is an explanatory diagram showing, in outline, the configuration of a modification of the cell-culturing apparatuses according to the first to third embodiments of the present invention.
{Fig. 13B} Fig. 13B is a side view of the cell-culturing apparatus in Fig. 13A.
{Fig. 13C} Fig. 13C is a side view of another example of the cell-culturing apparatus in Fig. 13A.
{Fig. 14A} Fig. 14A is an explanatory diagram showing, in outline, the configuration of a modification of the cell-culturing apparatuses according to the first to third embodiments of the present invention.
{Fig. 14B} Fig. 14B is an explanatory diagram showing, in outline, the configuration of another modification of the cell-culturing apparatuses according to the first to third embodiments of the present invention.
{Fig. 15A} Fig. 15A is an explanatory diagram showing, in outline, the configuration of a cell-culturing apparatus according to a fourth embodiment of the present invention.
{Fig. 15B} Fig. 15B is a diagram for explaining the operation of a closing member in the cell-culturing apparatus in Fig. 15A.
{Fig. 16A} Fig. 16A is an explanatory diagram showing, in outline, the configuration of the cell-culturing apparatus according to the fourth embodiment of the present invention.
{Fig. 16B} Fig. 16B is a front view of the cell-culturing apparatus in Fig. 16A.
{Fig. 17A} Fig. 17A is an explanatory diagram showing, in outline, the configuration of a modification of the cell-culturing apparatus according to the fourth embodiment of the present invention.
{Fig. 17B} Fig. 17B is a side view of the cell-culturing apparatus in Fig. 17A.
{Fig. 17C} Fig. 17C is a side view of another example of the cell-culturing apparatus in Fig. 17A.
{Fig. 18A} Fig. 18A is an explanatory diagram showing, in outline, the configuration of a modification of the cell-culturing apparatuses according to the first to fourth embodiments of the present invention.
{Fig. 18B} Fig. 18B is an explanatory diagram showing, in outline, the configuration of another modification of the cell-culturing apparatuses according to the first to fourth embodiments of the present invention.
{Fig. 19A} Fig. 19A is an explanatory diagram showing, in outline, the configuration of a cell-culturing apparatus according to a fifth embodiment of the present invention.
{Fig. 19B} Fig. 19B is a side view of the cell-culturing apparatus in Fig. 19A.
{Fig. 20A} Fig. 20A is an explanatory diagram showing, in outline, the configuration of an example closing means of the cell-culturing apparatus according to the fifth embodiment of the present invention.
{Fig. 20B} Fig. 20B is an explanatory diagram showing, in outline, the configuration of another example closing means of the cell-culturing apparatus according to the fifth embodiment of the present invention.
{Fig. 21A} Fig. 21A is an explanatory diagram showing, in outline, the configuration of an example closing means of the cell-culturing apparatus according to the fifth embodiment of the present invention.
{Fig. 21B} Fig. 21B is an explanatory diagram showing, in outline, the configuration of another example closing means of the cell-culturing apparatus according to the fifth embodiment of the present invention.
{Fig. 22A} Fig. 22A is an explanatory diagram showing, in outline, the configuration of an example driving means of the cell-culturing apparatus according to the fifth embodiment of the present invention.
{Fig. 22B} Fig. 22B is an explanatory diagram showing, in outline, the configuration of another example driving means of the cell-culturing apparatus according to the fifth embodiment of the present invention.
{Fig. 23A} Fig. 23A is an explanatory diagram (plan view) showing, in outline, the configuration of a modification of the cell-culturing apparatus according to the fifth embodiment of the present invention.
{Fig. 23B} Fig. 23B is a front view of the cell-culturing apparatus in Fig. 23A.
{Fig. 23C} Fig. 23C is a side view of the cell-culturing apparatus in Fig. 23A.
{Fig. 24A} Fig. 24A is an explanatory diagram (plan view) showing, in outline, the configuration of a modification of the cell-culturing apparatus according to the fifth embodiment of the present invention.
{Fig. 24B} Fig. 24B is a front view of the cell-culturing apparatus in Fig. 24A.
{Fig. 24C} Fig. 24C is a side view of the cell-culturing apparatus in Fig. 24A.
{Fig. 25A} Fig. 25A is an explanatory diagram showing, in outline, the configuration of a modification of the cell-culturing apparatus according to the fifth embodiment of the present invention.
{Fig. 25B} Fig. 25B is an explanatory diagram showing, in outline, the configuration of another modification of the cell-culturing apparatus according to the fifth embodiment of the present invention.
{Fig. 26} Fig. 26 is an explanatory diagram showing, in outline, the configuration of an example external door of the present invention.
{Fig. 27} Fig. 27 is an explanatory diagram showing, in outline, the configuration of a cell-culturing apparatus according to a sixth embodiment of the present invention.
{Fig. 28} Fig. 28 is an explanatory diagram showing, in outline, the configuration of a culturing unit of the cell-culturing apparatus according to the sixth embodiment of the present invention.
{Fig. 29} Fig. 29 is an explanatory diagram showing, in outline, the configurations of a gas-adjusting means and a gas-circulating means of the cell-culturing apparatus according to the sixth embodiment of the present invention.
{Fig. 30} Fig. 30 is an explanatory diagram showing, in outline, the configuration of a cell-culturing apparatus according to a seventh embodiment of the present invention.

### {Description of Embodiments}

### (First Embodiment)

A cell-culturing apparatus according to a first embodiment of the present invention will be described below with reference to the drawings.

A cell-culturing apparatus 100 according to this embodiment is an apparatus configured as shown in Figs. 1A and 1B and is provided with a culturing unit 1 that accommodates a culturing vessel 3 (cell-culturing vessel) and an incubator 2 that can accommodate a plurality of the culturing units 1.

The culturing unit 1 has a casing structure in which a plurality of shelf portions 4 that are arrayed in the vertical direction with spacings therebetween are provided in the interior thereof. More specifically, the culturing unit 1 has a box-shaped casing having a bottom surface and a top surface, which are arranged so as to be substantially parallel to each other with a spacing therebetween, and side surfaces, which connect the bottom surface and the top surface, and is installed so that the bottom surface of the casing becomes level. Therefore, in this specification, the direction parallel to the bottom surface is the "horizontal direction", and the direction orthogonal to the bottom surface is the "vertical direction". The interior space in the casing is an open space, and is configured so as to be the same environment as the culturing environment (temperature, humidity, CO₂ concentration, and so forth) outside the casing. The culturing vessels 3 can be placed on the shelf portions 4 in the casing interior.

With the incubator 2, it is possible to maintain the interior thereof at an environment that is appropriate for cell culturing, and it is possible to accommodate the plurality of culturing units 1 by arraying them in the horizontal direction.

By placing the culturing vessel 3 containing cells and a medium on the shelf portion 4 of the culturing unit 1 and by accommodating this culturing unit 1 in the incubator 2, it is possible to culture the cells in the culturing vessel 3.

The individual culturing units 1 can be moved into and out of the incubator 2 independently of each other. The incubator 2 is provided, in the side surface, with openings 5 through which the individual culturing units 1 can be moved into and out of the incubator 2, and the individual openings 5 are provided with closing means.

The closing means are configured so as to close the openings 5 in a state in which the culturing units 1 have been taken out of the incubator 2. Figs. 2A to 3B show examples of the closing means.

Fig. 2A shows, as the closing means, an example in which sliders 21 are installed so as to be movable in the top-to-bottom direction (vertical direction). When taking out the culturing units 1, the openings 5 can be opened by lifting the sliders 21 upward, and the culturing units 1 can be pulled out from the interior of the incubator 2 through the openings 5. The openings 5 are blocked by lowering the sliders 21 downward after pulling out the culturing units 1, and thus, it is possible to seal off the space in the incubator 2.

Fig. 2B shows, as the closing means, an example in which plate-like members 22 that rotate outward from the incubator 2 by using one side of each of the openings 5 as a rotating axis 23 are installed. When taking the culturing units 1 out, the openings 5 can be opened by pulling the plate-like members 22 toward the outside of the incubator 2, and the culturing units 1 can be pulled out of the interior of the incubator 2 through the openings 5. The openings 5 are blocked by returning the plate-like members 22 to the original positions after pulling out the culturing units 1, and thus, it is possible to seal off the space in the incubator 2.

Fig. 3A shows, as the closing means, an example in which plate-like members 31 that move into the interior of the incubator 2 by being pivoted inward into the incubator 2 by using one side of each of the openings as a rotating axis 32 are installed. In Fig. 3A, the top row shows a front view in which the incubator 2 is viewed from the openings 5 side, and the bottom row shows a plan view in which the incubator 2 is viewed from the top-surface side. The plate-like members 31 are configured so as to be disposed at positions at which the openings 5 are closed off by the plate-like members 31 in a state in which no external force is acting thereon. Therefore, in the state in which the culturing units 1 have been pulled out of the interior of the incubator 2, the openings 5 are blocked by the plate-like members 31, and thus, it is possible to seal off the space in the incubator 2.

When accommodating the culturing units 1 in the incubator 2, the plate-like members 31 are subjected to forces that act toward the interior of the incubator 2 by the culturing units 1 and are pivoted about the rotating axes 32, thus moving into the interior of the incubator 2. The culturing units 1 are provided with sealing members 33. When the culturing units 1 are completely pushed into the interior of the incubator 2, the sealing members 33 seal up the openings 5, and thus, it is possible to seal off the space in the incubator 2. Although Fig. 3A shows an example in which each opening 5 is closed up by one plate-like member 31, as shown in Fig. 3B, the sealing member 33 may seal off the opening 5 together with another plate-like member 35 that uses another side of the opening 5 as a rotating axis 34.

The closing means described above are examples, and it suffices that the closing means are configured so as to be capable of sealing off the openings 5 in the state in which the culturing units 1 have been taken out of the incubator 2 and of maintaining the internal environment in the incubator 2.

As shown in Fig. 4A, the incubator 2 may be provided with rail members 41 that extend in the direction in which the culturing units 1 are inserted into the incubator 2 for the purpose of positioning the culturing units 1. In this case, the culturing units 1 may be provided with locking members 42 for restricting the movements of the culturing units 1 in directions other than those along the rail members 41. The locking members 42 restrict the movements of the culturing units 1 in directions other than those along the rail members 41, for example, by interacting with the rail members 41 by fitting in groove portions formed by the rail members 41, as shown in Fig. 4A.

By doing so, the culturing units 1 can enter the interior of the incubator 2 along the rail members 41.

The incubator 2 may be provided, at the bottom surface that comes into contact with the culturing units 1 in the interior thereof, with movement facilitating means for making the movement of the culturing units 1 smooth. As shown in Fig. 4B, examples of the movement facilitating means include means in which a plurality of columnar members 43 are arrayed so as to be parallel to each other in the direction in which the culturing units 1 are moved (lateral direction in Fig. 4B). In Fig. 4A, the top row shows a diagram in which the columnar members 43 on the bottom surface of the incubator 2 are viewed from above, and the bottom row shows a diagram in which the columnar members 43 are viewed from the horizontal direction. By doing so, because the columnar members 43 are rotated in association with the movement of the culturing units 1, it is possible to smoothly move the culturing units 1.

Note that spherical members may be employed instead of the columnar members 43.

### (Second Embodiment)

A cell-culturing system according to a second embodiment of the present invention will be described below with reference to the drawings.

A cell-culturing system 200 according to this embodiment is a system configured as shown in Figs. 5A and 5B, and differs from the first embodiment in that a unit-moving mechanism for facilitating the movement of the culturing units 1 outside the incubator 2 is provided. The remaining configuration is the same as the first embodiment.

As shown in Figs. 5A and 5B, examples of the unit-moving mechanisms include rolling means 52 that facilitate movement of the culturing units 1 in the horizontal directions. The rolling means 52 are installed at the bottom surfaces of the culturing units 1 in a desired number (three or more) that makes it possible to support the culturing units 1. The rolling means 52 are provided with wheel-like members so that the culturing units 1 can be freely moved in both of the horizontal directions, and are secured to the bottom surfaces of the culturing units 1 via support members. Here, the wheel-like members may be ball-like members, and it suffices that the shape thereof allows the culturing units 1 to be moved in both of the horizontal directions.

Doing so facilities moving the culturing units 1 into and out of the incubator 2, and the culturing units 1 can be freely moved outside the incubator 2.

Next, another form of unit-moving mechanisms 51 will be described by using Figs. 6A and 6B. This form of the unit-moving mechanisms is formed of rolling means 53 for facilitating the movement of the culturing units 1 in the horizontal directions and housings 54 for accommodating the rolling means 53. The rolling means 53 are released from the housings 54 and secured when the culturing units 1 are pulled out of the incubator 2 to the outside. When the culturing units 1 are accommodated in the interior of the incubator 2, securing of the rolling means 53 is released, and the rolling means 53 are accommodated in the housings 54.

Fig. 7A shows an example of the rolling means 53.

The rolling means 53 is provided with a wheel-like member 55, a support member 56, and a stopper 57. The support member 56 is joined with the housing 54 at a fulcrum 58, and, by being substantially rotated about this fulcrum 58, the support member 56 is configured so as to allow the rolling means 53 to be moved into and out of the housing 54 (the fulcrum 58 is not rotated). An end of the support member 56 opposite from the fulcrum 58 is joined with the wheel-like member 55. The stopper 57 is installed in the support member 56, and is a member that stops the rotation of the rolling means 53 about the fulcrum 58.

Here, the wheel-like members 55 may be ball-like members, and it suffices that the shape thereof allows the culturing units 1 to be moved in both of the horizontal directions.

In addition, the range in which the rolling means 53 can be rotated about the fulcrum 58 may be restricted. For example, as shown in Fig. 7B, the movable range may be restricted at an angle of substantially 90°.

As shown in Figs. 8A and 8B, when the culturing units 1 are pulled out of the incubator 2, the rolling means 53 are rotated about the fulcrums 58 so as to follow arc-like tracks due to gravity exerted on the rolling means 53 themselves, thus being released from the housings 54. The rolling means 53 are substantially rotated about the fulcrums 58 until reaching predetermined positions (securing positions) at which the wheel-like members 55 reach the floor. The fulcrums 58 are designed so that the rolling means 53 are not rotated beyond the securing positions. When the rolling means 53 reach the securing positions, the stoppers 57 prevent the rolling means 53 from returning in the rotational direction about the fulcrums 58, thus securing the rolling means 53.

As shown in Figs. 8C and 8D, when the culturing units 1 are accommodated in the incubator 2, securing by the stoppers 57 is released by bringing securing-releasing means 59 installed in the incubator 2 into contact with the stoppers 57, and thus, the rolling means 53 are allowed to rotate about the fulcrums 58. When the culturing units 1 are pushed into the incubator 2 in this state, the rolling means 53 come into contact with the incubator 2 and are subjected to forces in the horizontal direction, and thus, the rolling means 53 are pushed up while substantially being rotated about the fulcrums 58 and are accommodated in the housings 54.

Regarding the method in which the stoppers secure the rolling means, although any method may be employed so long as the rolling means are secured so as not to allow rotation thereof about the fulcrums when the rolling means have reached the securing positions, the method may be configured as shown in, for example, Figs. 9A to 9C.

The stopper 57 is provided with a movable portion 57a and a secured portion 57b. The movable portion 57a is attached to the support member 56 via an elastic member (not shown), and is biased toward the fulcrum 58 by the elastic member. The movable portion 57a is configured to be movable in a direction d that connects the fulcrum 58 and the wheel-like member 55 against the elastic force of the elastic member when subjected to an external force. On the other hand, the secured portion 57b is secured to the fulcrum 58.

The movable portion 57a and the secured portion 57b can be placed in a state in which the movable portion 57a and the secured portion 57b are secured with each other depending on their mutual positional relationship. As shown in Figs. 9A to 9C, examples of the securing of the two components include a form in which the movable portion 57a is provided with a depression and the two components are secured when a portion of the secured portion 57b fits into the depression. In this case, at the predetermined position (securing position) at which the wheel-like member 55 reaches the floor, the movable portion 57a and the secured portion 57b are positioned so that the portion of the secured portion 57b fits into the depression on the movable portion 57a, thus being secured with each other.

In addition, the movable portion 57a is positioned on the support member 56 with the elastic member disposed therebetween, as described below. In the case in which external force in the direction away from the fulcrum 58 is acting on the movable portion 57a, the movable portion 57a is moved on the support member 56 away from the fulcrum 58 against the elastic force of the elastic member (state in Fig. 9C). In this state, because the movable portion 57a and the secured portion 57b are moved away from each other, securing of the two components is released. When an external force is not acting on the movable portion 57a, the movable portion 57a is moved on the support member 56 toward the fulcrum 58 due to the elastic force of the elastic member (state in Fig. 9A or 9B). The state in Fig. 9A is a state in which portion of the secured portion 57b fits into the depression on the movable portion 57a, thus securing the two components, and the state in Fig. 9B is a state in which the secured portion 57b is prevented from approaching the depression on the movable portion 57a by portion of the movable portion 57a.

When the culturing units 1 are pulled out of the incubator 2, the rolling means 53 are rotated about the fulcrums 58 so as to follow arc-like tracks due to gravity exerted on the rolling means 53 themselves, thus being released from the housings 54. The rolling means 53 are substantially rotated about the fulcrums 58 until reaching predetermined positions (securing positions) at which the wheel-like members 55 reach the floor, and thus, the secured portions 57b of the stoppers 57 installed at the fulcrums 58 collide with the movable portions 57a. At this time, the movable portions 57a are subjected to forces from the secured portions 57b, and are moved on the support members 56 away from the fulcrums 58 against the elastic forces of the elastic members, the secured portions 57b fit into the depressions on the movable portions 57a, and thus, the rolling means 53 are secured at the securing positions. At this time, the depressions on the movable portions 57a are subjected to forces in the direction in which the movable portions 57a would be returned to the original positions due to the elastic forces of the elastic members.

With regard to the method of releasing securing of the rolling means by using the securing-releasing means 59, any method may be employed so long as securing by the stoppers is released when the securing-releasing means installed in the incubator come into contact with the stoppers; for example, the method may be configured as shown in Figs. 10A and 10B.

The incubator 2 is provided with the securing-releasing means 59 at positions where the movable portions 57a of the stoppers 57 come into contact therewith when the culturing units 1 are accommodated. When the culturing units 1 are pushed into the incubator 2, the movable portions 57a are subjected to the forces from the securing-releasing means 59 are moved on the support members 56 in the direction away from the fulcrums 56 against the elastic forces of the elastic members, and the depressions on the movable portions 57a and the secured portions 57b are separated, thus achieving a state in which securing of the rolling means 53 is released. When the culturing units 1 are pushed into the incubator 2 in this state, the rolling means 53 come into contact with the incubator 2, receive forces in the horizontal direction, and are pushed up while substantially rotating about the fulcrums 58, thus being accommodated in the housings 54.

In this embodiment, although an example in which the movable portions of the stoppers are attached to the support members of the rolling means and the secured portions of the stoppers are attached to the fulcrums with respect to the housings has been described, the movable portions of the stoppers may be attached to the fulcrums with respect to the housings and the secured portions of the stoppers may be attached to the support members of the rolling means.

In other words, the stopper is constituted of the movable portion and the secured portion; one of the movable portion and the secured portion is attached to the fulcrum with respect to the housing and the other is attached to the support member of the rolling means; and the two components have shapes with which it is possible to secure each other depending on the relative positions between each other, and have the shapes with which it is possible to release securing between each other when being at relative positions other than the relative positions at which securing therebetween is achieved. Here, when the movable portion and the secured portion are placed at the relative positions at which they are secured to each other, the wheel-like member of the rolling means is secured at the predetermined position at which the wheel-like member reaches the floor.

### (Third Embodiment)

A cell-culturing system according to a third embodiment of the present invention will be described below with reference to the drawings.

A cell-culturing system 300 according to this embodiment is a system configured as shown in Figs. 11A and 11B, and differs from the individual embodiments described above in that the culturing unit 1 is provided with a solution supplying means 61 and a solution discharging means 62. The remaining configuration is the same as the individual embodiments described above.

The solution supplying means 61 is a means for supplying a medium to the culturing vessels 3 after holding the medium at a temperature that is appropriate for cell culturing (for example, 37 °C), and is provided with: a solution holding vessel 63 for holding the medium; tube-like members 64 that form medium flow channels that lead to the culturing vessels 3 from the solution holding vessel; and a supply-speed controlling means 65 for controlling the flow speed of the medium to be supplied to the culturing vessels 3.

The solution holding vessel 63 is provided with a discharge port 63a through which the medium is discharged, and this discharge port 63a is connected to the culturing vessels 3 via the tube-like members 64. As shown in Fig. 11A, the solution holding vessel 63 may be provided with a supply port 63b through which the medium is replenished from outside. By accommodating the culturing unit 1 in the incubator 2, it is possible to hold the temperature of the medium held therein at the same temperature as that of the interior of the incubator 2.

The tube-like members 64 may be made of a material having flexibility so that it is possible to change the direction of the flow channel; the tube-like members 64 may be made of a material having no flexibility with which the shape of the flow channel is fixed; or the tube-like members 64 may be formed by using a material having flexibility and a material having no flexibility in combination. One end of each tube-like member 64 is connected to the discharge port 63a of the solution holding vessel 63 and the other end thereof is connected to an opening (supply port 3a) in the culturing vessel 3.

The supply-speed controlling means 65 may be any means so long as it is possible to control the supply speed at which the medium is supplied to the culturing vessels 3. For example, the supply-speed controlling means 65 may be a feeding pump installed in the tube-like members 64, or may be a means for controlling the flow speed by adjusting the flow-channel diameter in the tube-like members 64. In the case in which the flow speed is controlled by adjusting the flow-channel diameter in the tube-like members 64, for example, it is permissible to employ a form in which the medium is made to flow in the tube-like members 64 due to gravity by disposing the solution holding vessel 63 above the culturing vessels 3 in the gravity direction or a form in which the medium is made to flow in the tube-like members 64 by a pressure exerted on the solution in the solution holding vessel 63.

The solution discharging means 62 is a means for discharging the medium from the culturing vessel 3 as a waste liquid, and is provided with a waste-liquid holding vessel 66 for holding the waste liquid; tube-like members 67 that form flow channels that connect the waste-liquid holding vessel 66 and the culturing vessels 3; and a discharge-speed controlling means 68 for controlling the flow speed of the waste liquid that flows through the tube-like members 67.

The waste-liquid holding vessel 66 is provided with a supply ports 66a that are connected to the tube-like members 67 and to which the waste liquid is supplied. As shown in Fig. 11B, the waste-liquid holding vessel 66 may be provided with a discharge port 66b for discharging the waste liquid to outside.

The tube-like members 67 may be made of a material having flexibility so that it is possible to change the direction of the flow channels; the tube-like members 67 may be made of a material having no flexibility with which the shapes of the flow channels are fixed; or the tube-like members 67 may be formed by using a material having flexibility and a material having no flexibility in combination. One end of each tube-like member 67 is connected to the supply port 66a in the waste-liquid holding vessel 66 and the other end thereof is connected to an opening (discharge port 3b) in the culturing vessel 3.

The discharge-speed controlling means 68 may be any means so long as it is possible to control the discharge speed at which the waste liquid is discharged from the culturing vessel 3 via the tube-like members 67. For example, the discharge-speed controlling means 68 may be a feeding pump installed in the tube-like members 67, or may be a means for exerting negative pressure to the waste-liquid holding vessel 66 and controlling the discharge speed by controlling the negative pressure.

In this embodiment, the solution supplying means 61, the solution discharging means 62, and the culturing vessel 3 are provided as a single unit in the culturing unit 1, and, by moving the culturing unit 1 into and out of the incubator 2, these components can be moved into and out of the incubator 2 as a single unit. Accordingly, when performing procedures such as replenishing the medium, discarding the waste liquid, etc. outside the incubator 2, these procedures can be efficiently performed by taking out the culturing medium 1.

In the individual embodiments described above, although the forms in which the culturing unit 1 is moved into and out of the incubator 2 from the side surface thereof have been described, for example, it is permissible to employ a form in which the culturing unit 1 is moved into and out of the incubator 2 from the bottom surface of the incubator 2, as shown in Fig. 12A, or a form in which the culturing unit 1 is moved into and out of the incubator 2 from the top surface of the incubator 2, as shown in Fig. 12B.

As shown in Figs. 13A and 13C, the individual embodiments described above may be provided with a driving means 6 for moving the incubator 2 in a horizontal direction h. With the driving means 6, it is possible to move the incubator 2 in the direction (horizontal direction h) in which the plurality of culturing units 1 are arrayed in the incubator 2. By doing so, when moving the culturing units 1 into and out of the incubator 2, by moving the incubator 2 with respect to a work table 7 by means of the driving means 6, the worker can move the culturing units 1 into and out of the incubator 2 by placing the openings 5 for desired culturing units 1 at positions at which the procedures are easily performed.

The worker may manually operate the driving means 6, or the worker may electrically operate the driving means 6 by turning ON/OFF a switch or the like. Fig. 13B shows an example in which the operation is manually performed by the worker, and is a form in which, when the worker rotates a rotating portion 8 of the driving means 6, the incubator 2 is moved in association therewith. Fig. 13C shows an example in which the operation is electrically performed, and is a form in which, when the worker steps on a pedal 9 of the driving means 6, the incubator 2 is moved in association therewith.

Although Figs. 13A to 13C show the forms in which the culturing unit 1 is moved into and out of the incubator 2 from the side surface thereof, for example, it is permissible to employ a form in which the culturing unit 1 is moved into and out of the incubator 2 from the top surface of the incubator 2, as shown in Fig. 14A, or a form in which the culturing unit 1 is moved into and out of the incubator 2 from the bottom surface of the incubator 2, as shown in Fig. 14B.

The work table 7 may be provided with rails (not shown) that guide the pulled-out culturing unit 1. In addition, the culturing unit 1 may be provided with casters at the bottom surface so that the culturing unit 1 can freely be moved in both of the horizontal directions.

Note that the arrangements of the driving means 6 shown in Figs. 13A to 14B are examples, and the arrangement thereof can be set, as appropriate, so long as the incubator 2 can be moved in the horizontal directions.

### (Fourth Embodiment)

A cell-culturing system according to a fourth embodiment of the present invention will be described below with reference to the drawings. A cell-culturing system 400 according to this embodiment is a system configured as shown in Figs. 15A and 15B, and differs from the first embodiment in that an incubator 72 accommodates a plurality of culturing units 71 by arraying them not only in the horizontal direction but also in the vertical direction. The remaining configuration is the same as the first embodiment.

The individual culturing units 71 can be moved into and out of the incubator 72 independently of each other. The incubator 72 is provided with openings 73 through which the individual culturing units 71 can be moved into and out of the incubator 72, and the individual openings 73 are provided with closing means.

The closing means are configured so as to close the openings 73 in a state in which the culturing units 71 have been taken out of the incubator 72.

In Fig. 15A, the top row shows a perspective view of the cell-culturing system 400, and the bottom row shows a front view of the cell-culturing system 400 in which the blocking means are omitted.

Fig. 15B shows, as the closing means, an example in which plate-like members 75 that rotate outward from the incubator 72 by using one side of each of the openings 73 as a rotating axis 74 are installed. When taking the culturing units 71 out, the openings 73 can be opened by pulling the plate-like members 75 toward the outside of the incubator 72, and the culturing units 71 can be pulled out of the interior of the incubator 72 through the openings 73. The openings 73 are blocked by returning the plate-like members 75 to the original positions after pulling out the culturing units 71, and thus, it is possible to seal off the space in the incubator 72.

Figs. 16A and 16B show, as the closing means, an example in which plate-like members 77 that move into the interior of the incubator 72 by being pivoted inward into the incubator 72 by using one side of each of the openings as a rotating axis 76 are installed.

The plate-like members 77 are configured so as to be disposed at positions at which the openings 73 are closed off by the plate-like members 77 in a state in which no external force is acting thereon. In the state in which the culturing units 71 have been pulled out of the interior of the incubator 72, the openings 73 are blocked by the plate-like members 77, and thus, it is possible to seal off the space in the incubator 72. When accommodating the culturing units 71 in the incubator 72, the plate-like members 77 are subjected to forces that act toward the interior of the incubator 72 by the culturing units 71 and are rotated about the rotating axes 76, thus moving into the interior of the incubator 72.

The culturing units 71 are provided with sealing members 78. When the culturing units 71 are completely pushed into the interior of the incubator 72, the sealing members 78 seal up the openings 73, and thus, it is possible to seal off the space in the incubator 72.

Although Figs. 16A and 16B show an example in which each opening 73 is closed up by using one plate-like member 77, the plate-like member 77 may seal off the opening 73 together with another plate-like member that uses another side of the opening 73 as a rotating axis.

The sealing means described above are examples, and it suffices that the closing means are configured so as to be capable of sealing off the openings 73 in the state in which the culturing units 71 have been taken out of the incubator 72 and of maintaining the internal environment of the incubator 72.

As shown in Figs. 17A to 17C, in this embodiment, a driving means 10 for moving the incubator 72 in a vertical direction p and a horizontal direction h may be provided. With the driving means 10, it is possible to move the incubator 72 in the directions (horizontal direction h and vertical direction p) in which the plurality of culturing units 71 are arrayed in the incubator 72. By doing so, when moving the culturing units 71 into and out of the incubator 72, by moving the incubator 72 with respect to a work table 11 by means of the driving means 10, the worker can move the culturing units 71 into and out of the incubator 72 by placing the openings 73 for desired culturing units 71 at positions at which the procedures are easily performed.

The worker may manually operate the driving means 10, or the worker may electrically operate the driving means 10 by turning ON/OFF a switch or the like. Fig. 17B shows an example in which the operation is manually performed by the worker, and is a form in which, when the worker rotates a rotating portion 12 of the driving means 10, the incubator 72 is moved in association therewith. Fig. 17C shows an example in which the operation is electrically performed, and is a form in which, when the worker steps on a pedal 13 of the driving means 10, the incubator 72 is moved in association therewith. As shown in the drawings, one each of the rotating portion and the pedal may be installed for horizontal movement and vertical movement.

The work table 11 may be provided with rails that guide the pulled-out culturing unit 71. In addition, the culturing unit 71 may be provided with casters at the bottom surface so that the culturing unit 71 can freely be moved in both of the horizontal directions.

Note that the arrangements of the driving means 10 shown in Figs. 17A to 17C are examples, and the arrangement thereof can be set, as appropriate, so long as the incubator 72 can be moved in the horizontal directions h and the vertical directions p.

In the individual embodiments described above, the incubator 2 may have a structure in which the culturing unit 1 is the only component a plurality of which are accommodated in the interior thereof, as shown in Fig. 18A, or the incubator 2 may be provided with a normal culturing space 81 that does not have a unitized structure together with the culturing unit 1, as shown in Fig. 18B.

### (Fifth Embodiment)

A cell-culturing system according to a fifth embodiment of the present invention will be described below with reference to the drawings.

A cell-culturing apparatus 500 according to this embodiment is an apparatus configured as shown in Figs. 19A and 19B, provided with: a culturing unit 91 that accommodates the culturing vessels 3; an incubator 92 that can accommodate a plurality of the culturing units 91; a driving means 93 for moving the incubator 92 in the vertical direction; and a work table 94.

The culturing unit 91 has a casing structure that can accommodate the culturing vessels 3 in the interior thereof. The interior space of the casing is an open space, and is configured so as to have the same environment as the culturing environment (temperature, humidity, CO₂ concentration, and so forth) outside the casing.

With the incubator 92, it is possible to maintain the interior thereof at an environment that is appropriate for cell culturing, and it is possible to accommodate the plurality of culturing units 91 by placing them next to each other in the vertical direction.

By placing the culturing vessels 3 containing cells and a medium in the culturing unit 91 and by accommodating this culturing unit 91 in the incubator 92, it is possible to culture the cells in the culturing vessels 3.

The individual culturing units 91 can be moved into and out of the incubator 92 independently of each other. The incubator 92 is provided with openings 95 through which the individual culturing units 91 can be moved into and out of the incubator 92, and the individual openings 95 are provided with closing means.

The closing means are configured so as to close the openings 95 in a state in which the culturing units 91 have been taken out of the incubator 92. Figs. 20A to 21B show examples of the closing means.

Fig. 20A shows, as the closing means, an example in which sliders 96 are installed so as to be movable in the left-to-right direction (horizontal direction). When taking out the culturing units 91, the openings 95 can be opened by moving the sliders 96 in the horizontal direction, and the culturing units 91 can be pulled out from the interior of the incubator 92 through the openings 95. The openings 95 are blocked by moving the sliders 96 in the horizontal direction after pulling out the culturing units 91, and thus, it is possible to seal off the space in the incubator 92.

Fig. 20B shows, as the closing means, an example in which plate-like members 97 that rotate outward from the incubator 92 by using one side (any one of sides) of each of the openings 95 as a rotating axis 107 are installed. When taking out the culturing units 91, the openings 95 can be opened by pulling the plate-like members 97 toward outside the incubator 92, and the culturing units 91 can be pulled out of the interior of the incubator 92 through the openings 95. The openings 95 are blocked by returning the plate-like members 97 to the original positions after pulling out the culturing units 91, and thus, it is possible to seal off the space in the incubator 92. In Fig. 20B, the top row shows a front view of the incubator 92, and the bottom row shows a side view of the incubator 92.

Fig. 21A shows, as the closing means, an example in which plate-like members 98a that move into the interior of the incubator 92 by being pivoted inward into the incubator 92 by using one side (any one of sides) of each of the openings 95 as a rotating axis 108 are installed. The plate-like members 98a are configured so as to be disposed at positions at which the openings 95 are closed off by the plate-like members 98a in a state in which no external force is acting thereon, and, in the state in which the culturing units 91 have been pulled out of the interior of the incubator 92, the openings 95 are blocked by the plate-like members 98a, and thus, it is possible to seal off the space in the incubator 92.

When accommodating the culturing units 91 in the incubator 92, the plate-like members 98a are subjected to forces that act toward the interior of the incubator 92 by the culturing units 91 and are rotated about the rotating axes 108, thus moving into the interior of the incubator; the culturing units 91 are provided with sealing members 99; and, when the culturing units 91 are completely pushed into the interior of the incubator 92, the sealing members 99 seal up the openings 95, and thus, it is possible to seal off the space in the incubator 92.

Although Fig. 21A shows an example in which each opening 95 is closed up by one plate-like member 98a, as shown in Fig. 21B, the plate-like member 98b may seal off the opening 95 together with another plate-like member 98a that uses another side of the opening 95 as a rotating axis 109. In Figs. 21A and 21B, the top rows show front views of the incubator 92, and the bottom rows show side views of the incubator 92.

The closing means described above are examples, and it suffices that the closing means are configured so as to be capable of sealing off the openings 95 in the state in which the culturing units 91 have been taken out of the incubator 92 and of maintaining the internal environment of the incubator 92.

The driving means 93 is a means for moving the incubator 92 in a vertical direction p, and is a means for moving the individual openings 95 corresponding to the individual culturing units 91 to the height at which the work table 94 is positioned. The worker may manually operate the driving means 93, or the worker may electrically operate the driving means 93 by turning on/off a switch or the like. Fig. 22A shows an example in which the operation is manually performed by the worker, and is a form in which, when the worker rotates a rotating portion 101, the incubator 92 is moved up and down in association therewith. Fig. 22B shows an example in which the operation is electrically performed, and is a form in which, when the worker steps on a pedal 102, the incubator 92 is moved up and down in association therewith.

It is preferable to provide a mechanism with which it is possible to perform positioning of the incubator 92 when the individual openings 95 of the incubator 92 have reached the height at which the work table 94 is positioned.

In the case in which the worker manually performs the operation, this mechanism may be configured so that, for example, resistance is applied to the hand of the worker operating the driving means 93 (sensation of clicking is transmitted to the hand, or the like) each time the individual openings 95 reach the height at which the work table 94 is positioned.

In the case in which the operation is electrically performed, this mechanism may be configured such that, for example, the closest opening 95 is positioned and stopped at the height at which the work table 94 is positioned when the worker turns on a switch, the next opening 95 is positioned at the height at which the work table 94 is positioned when the switch is turned on again, and the openings 95 are sequentially positioned at the height at which the work table 94 is positioned each time the worker turns on the switch. The mechanism may be configured such that a plurality of switches corresponding to the individual openings 95 are provided, and, when a switch corresponding to a desired opening 95 is turned on, the corresponding opening 95 is positioned at the height at which the work table 94 is positioned.

As shown in Figs. 23A to 23C, rails 103 that guide the culturing unit 91 may be installed in the work table 94.

Alternatively, as shown in Figs. 24A to 24C, the culturing unit 91 may be provided with casters 104 at the bottom surface so that the culturing unit 91 can be freely moved in both of the horizontal directions.

The incubator 92 may be provided with shelves 105 for placing the culturing units 91 thereon, as shown in Fig. 25A, or the incubator 92 may be provided with rails 106 for placing the culturing units 91 thereon, as shown in Fig. 25B.

In this case, movement facilitating means for making the movements of the culturing units 91 smooth may be provided on surfaces of the shelves 105 or the rails 106 that come into contact with the culturing units 91. Examples of the movement facilitating means include a means in which a plurality of columnar members or spherical members are arrayed so as to be parallel to each other in the direction in which the culturing units 91 are moved. By doing so, the columnar members or the spherical members are rotated in association with the movements of the culturing units 91, and thus, it is possible to smoothly move the culturing units 91.

In this embodiment, although a form in which the incubator is moved in the vertical direction with respect to the work table by means of the driving means has been described, it suffices that the incubator and the work table are moved relative to each other, and the driving means may move the work table in the vertical direction or the driving means may move the incubator and the work table together.

In the individual embodiments described above, as shown in Fig. 26, the incubator may be provided with, outside the closing means with which the openings for moving the individual culturing units into and out of the incubator are opened/closed, an external door 82 that covers the entirety of the openings for the individual culturing units.

In the individual embodiments described above, the operation of moving the culturing units into and out of the incubator may be manually performed by the worker or electrically performed by means of a driving means (not shown).

### (Sixth Embodiment)

A cell-culturing system according to a sixth embodiment of the present invention will be described below with reference to the drawings.

A cell-culturing apparatus 600 according to this embodiment is an apparatus configured as shown in Fig. 27 and provided with: a culturing unit 201 that accommodates a culturing vessel (cell-culturing vessel) 203; a gas-adjusting means 206 for adjusting the composition, temperature, or the like of a gas; a gas-flowing means 205 for making the gas flow between the gas-adjusting means 206 and the culturing unit 201; and an incubator 202 that can accommodate the culturing unit 201; and a work space 204.

As shown in Fig. 28, the culturing unit 201 has a casing structure that can accommodate the culturing vessels 203 in the interior thereof. The culturing unit 201 is provided with an opening 201c through which the culturing vessels 203 are moved into and out of the culturing unit 201 and a shutter 201d for opening/closing the opening 201c. In addition, the culturing unit 201 is provided with a supply port 201a for supplying the gas to the interior thereof and a discharge port 201b for discharging the gas to the outside. The left diagram, the middle diagram, and the right diagram in Fig. 28 are a back view, a side view, and a front view of the culturing unit 201, respectively.

When the culturing unit 201 is placed in the incubator 202, by setting the interior of the culturing unit 201 to be an open space by setting a state in which the opening 201c is open, it is possible to set the interior of the culturing unit 201 to be the same environment as that in the incubator 202.

When taking the culturing unit 201 out of the incubator 202, by setting the interior of the culturing unit 201 to be a closed space by setting a state in which the opening 201c is closed by the shutter 201d, it is possible to maintain the interior of the culturing unit 201 to be the same environment as that in the incubator 202.

The culturing unit 201 taken out of the incubator 202 is placed in the work space 204, and the worker performs various procedures. Examples of the work space 204 include an isolator, a clean bench, and so forth.

The gas-flowing means 205 is provided with: a main unit 205a; a gas supply pipe 205b; and a gas recovery pipe 205c.

The gas supply pipe 205b can be connected to the supply port 201a of the culturing unit 201, and can supply gas that has been adjusted by the gas-adjusting means 206 to the culturing unit 201 via the main unit 205a.

The gas recovery pipe 205c can be connected to the discharge port 201b of the culturing unit 201, and can feed the gas in the culturing unit 201 into the gas-adjusting means 206 via the main unit 205a.

The main unit 205a is a means for generating a flow of the gas so that the gas flows between the culturing unit 201 and the gas-adjusting means 206. The main unit 205a may be provided with, for example, a gas-discharging means for discharging the gas into the gas supply pipe 205b or a gas-sucking means for sucking out the gas from the gas recovery pipe 205c, or the main unit 205a may be provided with both of the gas-discharging means and the gas-sucking means.

The gas-adjusting means 206 is a means for adjusting composition, temperature, or the like of the gas, and is provided with a gas mixing means 206a and a temperature controlling means 206b, as shown in Fig. 29.

The gas mixing means 206a is connected to a CO₂ canister 205f, an O₂ canister 205d, and an N₂ canister 205e via tube-like members, and is a means for preparing a gas having a desired composition (for example, 5 % CO₂) by mixing appropriate amounts of gases from the respective canisters.

The temperature controlling means 206b can adjust the gas mixed by the gas mixing means 206a so as to reach a desired temperature (for example, 37 °C).

The thus-adjusted gas is supplied into the culturing unit 201 by the gas-flowing means 205 via the gas supply pipe 205b.

By repeating this step, it is possible to maintain a steady gas environment in the culturing unit 201 by circulating the gas between the culturing unit 201 and the gas-adjusting means 206.

Here, the temperature controlling means 206b of the gas-adjusting means 206 may be provided with a temperature sensor, a heater, and a controlling means, and the controlling means may control the heater while detecting the temperature by using the temperature sensor. Furthermore, the temperature controlling means 206b may be provided with a cooler, and, by controlling the heater and the cooler by using the controlling means, it is possible to perform control so as to more quickly achieve a desired temperature. PID control may be employed as the temperature control method.

The gas mixing means 206a of the gas-adjusting means 206 may be provided with a gas flowmeter and a controlling means, and the controlling means may control the gas mixing ratio while detecting flow volumes of the individual gases by using the gas flowmeter. Alternatively, with the gas mixing means 206a, the controlling means may control the gas mixing ratio by using gas cylinders. PID control may be employed as the gas-composition control method.

The gas-adjusting means 206 may be additionally provided with a means for adjusting the humidity of the gas (humidity controlling means). The humidity controlling means may be provided with a humidity sensor, a humidifier, and a controlling means, and the controlling means may control the humidifier while detecting the humidity by using the humidity sensor.

Next, example procedures of culturing cells by using the cell-culturing apparatus 600 according to this embodiment will be described.

The worker takes out the culturing unit 201 to the work space 204 after setting the opening 201c of the culturing unit 201 placed in the incubator 202 to a state in which the opening 201c is closed by the shutter 201d.

Next, in the work space 204, the worker connects the supply port 201a and the discharge port 201b of the culturing unit 201 to the gas supply pipe 205b and the gas recovery pipe 205c, respectively. Here, it is preferable that the supply port 201a and the discharge port 201b have structures that keep them in a closed state in a state in which the gas supply pipe 205b and the gas recovery pipe 205c are not connected thereto, and that open them when the gas supply pipe 205b and the gas recovery pipe 205c are connected thereto. By doing so, the gas is circulated between the culturing unit 201 and the gas-adjusting means 206, and thus, it is possible to maintain the environment in the culturing unit 201 in a desired state.

In this state, the worker performs procedures by moving a desired culturing vessel 203 into and out of the culturing unit 201 at a desired timing.

After completing the procedures, the gas supply pipe 205b and the gas recovery pipe 205c are removed from the supply port 201a and the discharge port 201b of the culturing unit 201, respectively. The culturing unit 201 is accommodated in the incubator 202, and the shutter 201d is retracted from the opening 201c of the culturing unit 201, thus setting the culturing unit 201 to be an open space.

Although the positions at which the supply port 201a and the discharge port 201b are installed in the culturing unit 201 are arbitrary, gas exchange efficiency is improved by installing them away from each other.

As the gas-adjusting means 206, a form in which only the CO₂ canister 205f is provided and the O₂ concentration and the N₂ concentration are not adjusted may be employed.

In addition, as the gas-adjusting means 206, a form in which the gas composition is not adjusted and only the gas temperature is adjusted may be employed.

In this embodiment, in the case in which the gas-adjusting means 206 adjusts the internal environment of the culturing unit 201 so as to be the same gas environment as that in the incubator 202 (for example, temperature of 37 °C, 5 % CO₂ concentration, and 95 % humidity), the incubator 202 may be omitted. In this case, the supply port 201a and the discharge port 201b of the culturing unit 201 should be continuously connected to the gas supply pipe 205b and the gas recovery pipe 205c, respectively.

As the culturing unit 201 of this embodiment, it is possible to employ the culturing units of the first to fifth embodiments. When doing so, the respective culturing units may be configured such that it is possible to form a closed space provided with a supply port for supplying gas to the interior thereof and a discharge port for discharging the gas to the outside, and that it is possible to set the interior thereof to be an open space or a closed space by means of a shutter.

Here, it is preferable that the supply port and the discharge port have structures that keep them in a closed state in a state in which the gas supply pipe and the gas recovery pipe are not connected thereto, and that open them when the gas supply pipe and the gas recovery pipe are connected thereto.

In this embodiment, although a form in which gas is circulated between the culturing unit 201 and the gas-adjusting means 206 by means of the gas-flowing means 205 has been described, it is permissible that the gas-flowing means 205 is not provided with the gas recovery pipe 205c and just supplies the culturing unit 201 with the gas adjusted by the gas-adjusting means 206. In this case, excess gas in the culturing unit 201 may be discharged, for example, from the discharge port 201b of the culturing unit 201.

### (Seventh Embodiment)

A cell-culturing system according to a seventh embodiment of the present invention will be described with reference to the drawings.

A cell-culturing apparatus 700 according to this embodiment is an apparatus configured as shown in Fig. 30, and is a cell-culturing apparatus provided with a gas-flowing means 207 instead of the gas-adjusting means 206 and the gas-flowing means 205 of the sixth embodiment. The rest of configurations are the same as those of the sixth embodiment.

The gas-flowing means 207 is a means that is communicated with the space in the incubator 202 and that is for making gas in the incubator 202 flow between the culturing unit 201 and the incubator 202. The gas-flowing means 207 is provided with: a main unit 207a; a gas supply pipe 207b; and a gas recovery pipe 207c.

The gas supply pipe 207b can be connected to the supply port 201a of the culturing unit 201, and can supply the gas in the incubator 202 to the culturing unit 201 via the main unit 207a.

The gas recovery pipe 207c can be connected to the discharge port 201b of the culturing unit 201, and can feed the gas in the culturing unit 201 into the incubator 202 via the main unit 207a.

The main unit 207a is a means for generating a gas flow so that the gas flows between the culturing unit 201 and the incubator 202. The main unit 207a may be provided with, for example, a gas-discharging means for discharging the gas into the gas supply pipe 207b or a gas-sucking means for sucking out the gas from the gas recovery pipe 207c, or the main unit 207a may be provided with both of the gas-discharging means and the gas-sucking means.

The procedures of culturing cells by using the cell-culturing apparatus 700 according to this embodiment are the same as those of the sixth embodiment.

As the culturing unit 201 of this embodiment, it is possible to employ the culturing units of the first to fifth embodiments. When doing so, the respective culturing units may be configured such that it is possible to form a closed space provided with a supply port for supplying gas to the interior thereof and a discharge port for discharging the gas to the outside, and that it is possible to set the interior thereof to be an open space or a closed space by means of a shutter.

Here, it is preferable that the supply port and the discharge port have structures that keep them in a closed state in a state in which the gas supply pipe and the gas recovery pipe are not connected thereto, and that open them when the gas supply pipe and the gas recovery pipe are connected thereto.

In this embodiment, although a form in which gas is circulated between the culturing unit 201 and the incubator 202 by means of the gas-flowing means 207 has been described, it is permissible that the gas-flowing means 207 is not provided with the gas recovery pipe 207c and just supplies the culturing unit 201 with the gas in the incubator 202. In this case, excess gas in the culturing unit 201 may be discharged, for example, from the discharge port 201b of the culturing unit 201.

Examples of the controlling means of the present invention include a personal computer (PC). For example, a PC may include a CPU and a memory, and may realize the function of the controlling means by causing the CPU to execute a control program stored in the memory.

### {Reference Signs List}

1, 71, 91, 201 culturing unit
2, 72, 92, 202 incubator
3, 203 culturing vessel (cell-culturing vessel)
4 shelf portion
5, 73, 95 opening
6, 10, 93 driving means
7, 11, 94 work table
8, 12, 101 rotating portion
9, 13, 102 pedal
21, 96 slider
22, 31, 35, 75, 77, 98a, 98b plate-like member
33, 78, 99 sealing member
41 rail member
42 locking member
43 columnar member
52, 53 rolling means
54 housing
55 wheel-like member
56 support member
57 stopper
58 fulcrum
59 securing-releasing means
61 solution supplying means
62 solution discharging means
63 solution holding vessel
64, 67 tube-like member
65 supply-speed controlling means
66 waste-liquid holding vessel
68 discharge-speed controlling means
82 external door
103 rail
104 caster
105 shelf
106 rail
204 work space
205, 207 gas-flowing means
205a, 207a main unit
205b, 207b gas supply pipe
205c, 207c gas recovery pipe
206a gas mixing means
206b temperature controlling means

## Claims

1. A cell-culturing apparatus comprising:
a culturing unit in which a culturing vessel is placed; and
an incubator in which a plurality of the culturing units are placed in an interior thereof, and that can maintain the interior thereof at an environment that is appropriate for cell culturing,
wherein the incubator includes
openings that are provided for each of the plurality of culturing units and through which the culturing units can separately be moved into and out of the incubator, and
closing means that are provided at the openings and that seal off the internal environment of the incubator from outside when the culturing units are taken out of the incubator.

2. A cell-culturing apparatus according to Claim 1, further comprising: a driving means that moves the incubator.

3. A cell-culturing apparatus according to Claim 2, wherein the driving means can move the incubator in a direction in which the plurality of culturing units are arrayed in the interior of the incubator.

4. A cell-culturing apparatus according to any one of Claims 1 to 3, wherein the culturing unit is provided with a caster.

5. A cell-culturing apparatus according to any one of Claims 1 to 4, wherein the culturing unit is provided with a solution supplying means that supplies a solution such as a medium or the like to the culturing vessel and a solution discharging means that discharges a waste liquid from the culturing vessel.

6. A cell-culturing apparatus according to any one of Claims 1 to 4, wherein the plurality of culturing units are arrayed in the interior of the incubator in a horizontal direction.

7. A cell-culturing apparatus according to any one of Claims 1 to 4, wherein the plurality of culturing units are arrayed in the interior of the incubator in a vertical direction.

8. A cell-culturing apparatus according to any one of Claims 1 to 4, wherein the plurality of culturing units are arrayed in the interior of the incubator in a horizontal direction and a vertical direction.

9. A cell-culturing apparatus comprising:
a culturing unit in which a culturing vessel is placed;
an incubator in which the culturing unit is placed in an interior thereof, and that can maintain the interior thereof at an environment that is appropriate for cell culturing;
a work space in which the culturing unit taken out of the incubator is placed;
a gas-adjusting means that adjusts at least one of gas composition, gas temperature, and gas humidity; and
a gas-flowing means that makes a gas flow between the culturing unit placed in the work space and the gas-adjusting means.

10. A cell-culturing apparatus according to Claim 9, wherein the incubator serves as the gas-adjusting means.

11. A cell-culturing apparatus according to Claim 9 or 10, wherein the culturing unit forms an open space in the culturing unit when being placed in the interior of the incubator, and forms a closed space in the culturing unit when being placed in the interior of the work space.

12. A cell-culturing apparatus according to any one of Claims 9 to 11, wherein the gas-flowing means makes gas circulate between the culturing unit placed in the work space and the gas-adjusting means.
